# EUROPÄISCHE PATENTSCHRIFT

(11) **EP 0 740 660 B1**
(45) Veröffentlichungstag und Bekanntmachung des Hinweises auf die Patenterteilung: **07.11.2001**
(21) Anmeldenummer: 95905118.6
(22) Anmeldetag: 04.01.1995
(51) Int. Cl.: C07D 263/10, A01N 43/76, C07D 263/14, C07D 263/12

(54) **SUBSTITUIERTE OXAZOLINE**
SUBSTITUTED OXAZOLINES
OXAZOLINES SUBSTITUEES

(30) Priorität: 17.01.1994 DE 4401099
(43) Veröffentlichungstag der Anmeldung: 06.11.1996
(60) Teilanmeldung: 01109541.1
(73) Patentinhaber: BAYER AG, 51368 Leverkusen (DE)
(72) Erfinder: KRÄMER, Wolfgang, D-51399 Burscheid (DE); FISCHER, Reiner, D-40789 Monheim (DE); HOLMWOOD, Graham, D-42327 Wuppertal (DE); HAGEMANN, Hermann, D-51375 Leverkusen (DE); WACHENDORFF-NEUMANN, Ulrike, D-40789 Monheim (DE); ERDELEN, Christoph, D-42799 Leichlingen (DE); TURBERG, Andreas, D-40699 Erkrath (DE); MENCKE, Norbert, D-51381 Leverkusen (DE)
(86) Internationale Anmeldenummer: EP9500021
(87) Internationale Veröffentlichungsnummer: WO9519349

(56) Entgegenhaltungen:
- EP-A- 0 345 775
- EP-A- 0 432 661
- EP-A- 0 553 623
- CHEMICAL ABSTRACTS, vol. 120, no. 21, 23. Mai 1994, Columbus, Ohio, US; abstract no. 270196a, Seite 1049 ; & TETRAHEDRON LETT., Bd.34, Nr.48, 1993 Seiten 7793 - 7796
- CHEMICAL ABSTRACTS, vol. 120, no. 17, 25. April 1994, Columbus, Ohio, US; abstract no. 217380e, Seite 1049 ; & TETRAHEDRON LETT., Bd.34, Nr.38, 1993 Seiten 5983 - 5986
- CHEMICAL ABSTRACTS, vol. 116, no. 25, 22. Juni 1992, Columbus, Ohio, US; abstract no. 255223f, Seite 749 ; & TETRAHEDRON LETT., Bd.33, Nr.13, 1992 Seiten 1743 - 1746
- CHEMICAL ABSTRACTS, vol. 116, no. 9, 2. März 1992, Columbus, Ohio, US; abstract no. 83659j, Seite 820 ; & JP,A,3 197 469 (NIPPON MINING CO.)
- CHEMICAL ABSTRACTS, vol. 114, no. 1, 7. Januar 1991, Columbus, Ohio, US; Seite 798 ; & JP,A,2 235 872 (NIPPON MINING CO.)
- CHEMICAL ABSTRACTS, vol. 112, no. 17, 23. April 1990, Columbus, Ohio, US; abstract no. 158234v, Seite 718 ; & JP,A,1 228 972 (INSTITUTE OF PHYSICAL AND CHEMICAL RESEARCH)
- CHEMICAL ABSTRACTS, vol. 102, no. 5, 4. Februar 1985, Columbus, Ohio, US; abstract no. 45822, Seite 565 ; & J. AM. CHEM. SOC., Bd.107, Nr.2, 1985 Seiten 443 - 448
- CHEMICAL ABSTRACTS, vol. 102, no. 15, 15. April 1985, Columbus, Ohio, US; abstract no. 131336m, Seite 568 ; & BULL. CHEM. SOC. JPN., Bd.57, Nr.12, 1984 Seiten 3531 - 3535
- CHEMICAL ABSTRACTS, vol. 98, no. 13, 28. März 1983, Columbus, Ohio, US; abstract no. 106914q, Seite 566 ; & BULL. CHEM. SOC. JPN., Bd.55, Nr.11, 1982 Seiten 3546 - 3551
- CHEMICAL ABSTRACTS, vol. 97, no. 13, 27. September 1982, Columbus, Ohio, US; abstract no. 109704, Seite 581 ; & JP,A,8 258 641 (JAPAN TOBACCO AND SALT PUBLIC CORP.)
- CHEMICAL ABSTRACTS, vol. 96, no. 13, 29. März 1982, Columbus, Ohio, US; abstract no. 103579g, Seite 648 ; & HETEROCYCLES, Bd.16, Nr.11, 1981 Seiten 1901 - 1905
- CHEMICAL ABSTRACTS, vol. 94, no. 3, 19. Januar 1981, Columbus, Ohio, US; abstract no. 15626y, Seite 432 ; & CAN. J. CHEM., Bd.58, Nr.23, 1980 Seiten 2562 - 2566

## Beschreibung

Die Erfindung betrifft die Verwendung substituierter Oxazoline zur Bekämpfung tierischer Schädlinge, neue substituierte Oxazoline und mehrere Verfahren zu ihrer Herstellung.

Es ist bereits bekannt, daß bestimmte Oxazolin-Derivate insektizide und akarizide Eigenschaften aufweisen (vgl. EP-A 0 345 775 und EP-A 0 432 661). Die Wirksamkeit dieser vorbekannten Verbindungen ist jedoch, insbesondere bei niedrigen Aufwandmengen und Konzentrationen nicht in allen Anwendungsgebieten völlig zufriedenstellend.

Gefunden wurde die Verwendung substituierter Oxazoline der Formel (I) 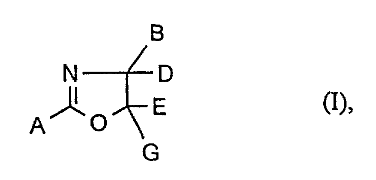 in welcher
- A: für C₁-C₈-Alkyl, das gegebenenfalls einfach oder mehrfach, gleich oder verschieden substituiert ist duch Halogen, Cyano, C₁-C₄-Alkoxy sowie durch jeweils gegebenenfalls einfach bis dreifach, gleich oder verschieden durch Halogen, C₁-C₄-Alkyl, C₁-C₄-Halogenalkyl, C₁-C₄-Alkoxy, C₁-C₄-Halogenalkoxy, C₁-C₄-Alkylthio und/oder C₁-C₄-Halogenalkylthio substituiertes Phenyl, Phenoxy, Benzyloxy, Phenylthio und Benzylthio,
für gegebenenfalls einfach oder mehrfach, gleich oder verschieden durch Halogen substituiertes C₂-C₈-Alkenyl,
für C₃-C₈-Cycloalkyl, das gegebenenfalls einfach oder mehrfach, gleich oder verschieden substituiert ist durch
Halogen, C₁-C₄-Alkyl, C₂-C₄-Alkenyl, C₁-C₄-Halogenalkyl oder C₂-C₄-Halogenalkenyl,
sowie für C₅-C₇-Cycloalkenyl steht, das gegebenenfalls einfach oder mehrfach, gleich oder verschieden substituiert ist durch Halogen, C₁-C₄-Alkyl oder C₁-C₄-Halogenalkyl und
- B: für jeweils gegebenenfalls einfach bis vierfach, gleich oder verschieden substituiertes Phenyl, Benzyl Pheneth-1-yl, Pheneth-2-yl, Phenoxymethyl, Phenylthiomethyl, Phenoxyeth-1-yl, Phenylthioeth-1-yl, Phenoxyeth-2-yl oder Styryl steht, wobei jeweils als Phenylsubstituenten genannt seien Halogen,
C₁-C₁₈-Alkyl,
C₁-C₈-Alkoxy-C₁-C₈-alkyl,
C₁-C₈-Halogenalkoxy,
C₁-C₄-Halogenalkyl,
C₁-C₁₈-Alkoxy, das gegebenenfalls durch weitere 1-3 Sauerstoffatome unterbrochen ist,
C₁-C₁₈-Alkylthio,
C₁-C₈-Halogenalkylthio,
3,4-Difluormethylendioxo,
3,4-Tetrafluorethylendioxo,
gegebenenfalls durch C₁-C₄-Alkyl, C₃-C₆-Cycloalkyl und/oder Halogen substituiertes Benzyliminooxymethyl,
jeweils gegebenenfalls durch C₁-C₆-Alkyl, C₁-C₆-Alkoxy, Cyclohexyl oder Phenyl substituiertes Cyclohexyl und Cyclohexyloxy;
gegebenenfalls einfach oder zweifach, gleich oder verschieden durch Halogen,
C₁-C₄-Alkyl oder C₁-C₄₋Halogenalkyl substituiertes Pyridyloxy; jeweils gegebenenfalls einfach bis dreifach, gleich oder verschieden durch C₁-C₁₂-Alkyl, Halogen, C₁-C₄-Halogenalkyl, C₁-C₆-Alkoxy, C₁-C₆--Halogenalkoxy, C₁-C₆-Alkoxy-C₁-C₆-alkyl, C₁-C₆-Alkoxy-ethylenoxy, C₁-C₆-Alkylthio und/oder C₁-C₆-Halogenalkylthio substituiertes Phenyl, Benzyl, Phenoxy, Phenylthio, Benzyloxy und Benzylthio,
- D: für Wasserstoff oder Methyl steht;
- E: für Wasserstoff oder Methyl steht; und
- G: für Wasserstoff oder Methyl steht
zur Bekämpfung von tierischen Schädlingen.

Weiterhin wurde gefunden, daß substituierte Oxazoline der Formel (I) sehr gut zur Bekämpfung von tierischen Schädlingen geeignet sind. Sie zeichnen sich insbesondere durch hohe Wirksamkeit gegen Arthropoden und Nematoden aus.

Überraschenderweise zeigen die erfindungsgemäßen, substituierten Oxazoline der Formel (I) eine erheblich bessere Wirksamkeit gegenüber tierischen Schädlingen als die konstitutionell ähnlichsten vorbekannten Verbindungen.

Bevorzugt verwendet werden Verbindungen der Formel (I),
in welcher
- A: für C₁-C₆-Alkyl, das gegebenenfalls einfach oder mehrfach, gleich oder verschieden substituiert ist durch
Fluor, Chlor, Brom, Cyano, C₁-C₂-Alkoxy sowie durch jeweils gegebenenfalls einfach oder zweifach, gleich oder verschieden durch Fluor, Chlor, C₁-C₄-Alkyl, C₁-C₂-Halogenalkyl, C₁-C₂-Alkoxy, C₁-C₂-Halogenalkoxy, C₁-C₂-Alkylthio und/oder C₁-C₂-Halogenalkylthio substituiertes Phenyl, Phenoxy, Benzyloxy, Phenylthio und Benzylthio;
für gegebenenfalls einfach oder mehrfach, gleich oder verschieden durch Fluor, Chlor und/oder Brom substituiertes C₂-C₆-Alkenyl;
für C₃-C₈-Cycloalkyl, das gegebenenfalls einfach oder mehrfach, gleich oder verschieden substituiert ist durch
Fluor, Chlor, C₁-C₄-Alkyl, C₂-C₄-Alkenyl, sowie jeweils durch Fluor und/oder Chlor substituiertes C₁-C₂-Alkyl oder C₂-C₄-Alkenyl;
sowie für C₅-C₇-Cycloalkenyl steht, das gegebenenfalls einfach oder mehrfach, gleich oder verschieden substituiert ist durch
Fluor, Chlor, C₁-C₄-Alkyl sowie durch Fluor und/oder Chlor substituiertes C₁-C₂-Alkyl und
- B: für jeweils gegebenenfalls einfach bis vierfach, gleich oder verschieden substituiertes Phenyl, Benzyl, Pheneth-1-yl, Pheneth-2-yl, Phenoxymethyl, Phenylthiomethyl, Phenoxyeth-1-yl, Phenoxyeth-2-yl, Phenylthioeth-1-yl oder Styryl steht,
wobei jeweils als Phenylsubstituenten genannt seien
F, Cl, Br,
C₁-C₁₈-Alkyl,
C₁-C₆-Alkoxy-C₁-C₈-alkyl,
einfach bis sechsfach, gleich oder verschieden durch F und/oder Cl substituiertes C₁-C₈-Alkoxy,
einfach bis fünffach, gleich oder verschieden durch F und/oder Cl substituiertes C₁-C₂-Alkyl,
C₁-C₁₈-Alkoxy und -(OC₂H₄)₁₋₃-O-C₁-C₆-alkyl,
C₁-C₁₂-Alkylthio,
einfach bis sechsfach, gleich oder verschieden durch F und/oder Cl substituiertes C₁-C₈-Alkylthio,
3,4-Difluormethylendioxo,
3,4-Tetrafluorethylendioxo,
die Gruppierungen 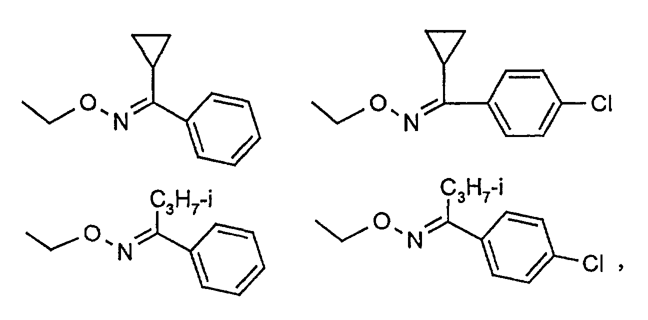 jeweils gegebenenfalls durch C₁-C₄-Alkyl, C₁-C₄-Alkoxy, Cyclohexyl oder Phenyl substituiertes Cyclohexyl und Cyclohexyloxy;
gegebenenfalls einfach oder zweifach, gleich oder verschieden durch F, Cl oder CF₃ substituiertes Pyridyloxy;
jeweils gegebenenfalls einfach bis dreifach, gleich oder verschieden durch C₁-C₁₂-Alkyl, F, Cl, Br, CF₃, C₁-C₄-Alkoxy, einfach bis sechsfach, gleich oder verschieden durch F und/oder Cl substituiertes C₁-C₄-Alkoxy, C₁-C₄-Alkoxy-C₁-C₄-alkyl, C₁-C₄-Alkoxy-ethylenoxy, C₁-C₄-Alkylthio, einfach bis sechsfach, gleich oder verschieden durch F und/oder Cl substituiertes C₁-C₄-Alkylthio substituiertes Phenyl, Benzyl, Phenoxy, Phenylthio, Benzyloxy und Benzylthio,
- D: für Wasserstoff oder Methyl steht;
- E: für Wasserstoff oder Methyl steht; und
- G: für Wasserstoff oder Methyl steht
zur Bekämpfung von tierischen Schädlingen.

Es wurden die neuen substituierten Oxazoline der Formel (Ia) gefunden, 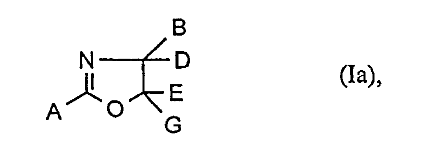 in welcher
- A: für C₁-C₈-Alkyl, das gegebenenfalls einfach oder mehrfach, gleich oder verschieden substituiert ist duch Halogen, Cyano, C₁-C₄-Alkoxy sowie durch jeweils gegebenenfalls einfach bis dreifach, gleich oder verschieden durch Halogen, C₁-C₄-Alkyl, C₁-C₄-Halogenalkyl, C₁-C₄-Alkoxy, C₁-C₄-Halogenalkoxy, C₁-C₄-Alkylthio und/oder C₁-C₄-Halogenalkylthio substituiertes Phenyl, Phenoxy, Benzyloxy, Phenylthio und Benzylthio,
für gegebenenfalls einfach oder mehrfach, gleich oder verschieden durch Halogen substituiertes C₂-C₈-Alkenyl,
für C₃-C₈-Cycloalkyl, das gegebenenfalls einfach oder mehrfach, gleich oder verschieden substituiert ist durch
Halogen, C₁-C₄-Alkyl, C₂-C₄-Alkenyl, C₁-C₄-Halogenalkyl oder C₂-C₄-Halogenalkenyl,
sowie für C₅-C₇-Cycloalkenyl steht, das gegebenenfalls einfach oder mehrfach, gleich oder verschieden substituiert ist durch
Halogen, C₁-C₄-Alkyl oder C₁-C₄-Halogenalkyl und
- B: für jeweils einfach bis vierfach, gleich oder verschieden substituiertes Phenyl, Benzyl Pheneth-1-yl, Pheneth-2-yl, Phenoxymethyl, Phenylthiomethyl, Phenoxyeth-1-yl, Phenylthioeth-1-yl, Phenoxyeth-2-yl oder Styryl steht, wobei jeweils als Phenylsubstituenten genannt seien Halogen,
C₁-C₁₈-Alkyl,
C₁-C₈-Alkoxy-C₁-C₈-alkyl,
C₁-C₈-Halogenalkoxy,
C₁-C₄-Halogenalkyl,
C₁-C₁₈-Alkoxy, das gegebenenfalls durch weitere 1-3 Sauerstoffatome unterbrochen ist,
C₁-C₁₈-Alkylthio,
C₁-C₈-Halogenalkylthio,
3,4-Difluormethylendioxo,
3,4-Tetrafluorethylendioxo,
gegebenenfalls durch C₁-C₄-Alkyl, C₃-C₆-Cycloalkyl und/oder Halogen substituiertes Benzyliminooxymethyl,
jeweils gegebenenfalls durch C₁-C₆-Alkyl, C₁-C₆-Alkoxy, Cyclohexyl oder Phenyl substituiertes Cyclohexyl und Cyclohexyloxy;
gegebenenfalls einfach oder zweifach, gleich oder verschieden durch Halogen,
C₁-C₄-Alkyl oder C₁-C₄₋Halogenalkyl substituiertes Pyridyloxy;
jeweils einfach bis dreifach, gleich oder verschieden durch C₁-C₁₂-Alkyl, Halogen, C₁-C₄-Halogenalkyl, C₁-C₆-Alkoxy, C₁-C₆-Halogenalkoxy, C₁-C₆-Alkoxy-C₁-C₆-alkyl, C₁-C₆-Alkoxy-ethylenoxy, C₁-C₆-Alkylthio und/oder C₁-C₆-Halogenalkylthio substituiertes Phenoxy, Phenylthio, Benzyloxy und Benzylthio,
- D: für Wasserstoff oder Methyl steht;
- E: für Wasserstoff oder Methyl steht; und
- G: für Wasserstoff oder Methyl steht.

Die Verbindungen der Formel (Ia) können auch in Abhängigkeit von der Art der Substituenten als geometrische und/oder optische Isomere oder Isomerengemische unterschiedlicher Zusammensetzung vorliegen. Die Erfindung betrifft sowohl die reinen Isomeren als auch die Isomerengemische.

Weiterhin wurde gefunden, daß man substituierte Oxazoline der Formel (Ia) erhält, wenn man
a) Aminoalkohole der Formel (II) 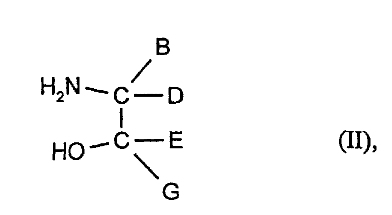 in welcher
   B, D, E und G die oben angegebenen Bedeutungen haben,
   mit einer Carbonsäure der Formel (III)

   A-COOH (III),

   in welcher
   - A: die oben angegebene Bedeutung hat,
   mit einem wasser-entziehenden Mittel und gegebenenfalls in Gegenwart eines Verdünnungsmittels umsetzt;
   oder
b) Amidalkohole der Formel (IV) 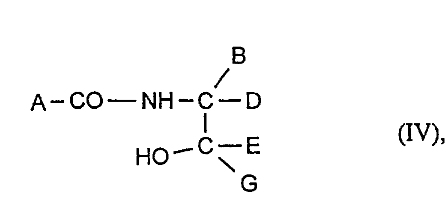 in welcher
   A, B, D, E und G die oben angegebenen Bedeutungen haben,
   mit einem wasser-entziehenden Mittel, gegebenenfalls in Gegenwart eines Verdünnungsmittels umsetzt;
   oder
c) Amid-Derivate der Formel (V) 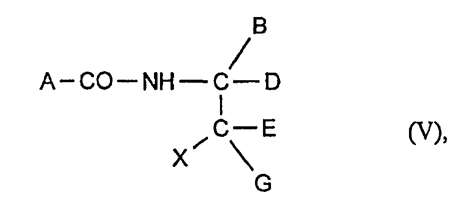 in welcher
   - A, B, D, E und G: die oben angegebenen Bedeutungen haben; und
   - X: für eine Abgangsgruppe, wie Halogen, Alkylsulfonyloxy oder Arylsulfonyloxy steht,
   mit einer Base, gegebenenfalls in Gegenwart eines Verdünnungsmittels umsetzt.

Die erfindungsgemäßen Verbindungen sind durch die Formel (Ia) allgemein definiert.
- A: steht bevorzugt für C₁-C₆-Alkyl, das gegebenenfalls einfach oder mehrfach, gleich oder verschieden substituiert ist durch
Fluor, Chlor, Brom, Cyano, C₁-C₂-Alkoxy sowie durch jeweils gegebenenfalls einfach oder zweifach, gleich oder verschieden durch Fluor, Chlor, C₁-C₄-Alkyl, C₁-C₂-Halogenalkyl, C₁-C₂-Alkoxy, C₁-C₂-Halogenalkoxy, C₁-C₂-Alkylthio und/oder C₁-C₂-Halogenalkylthio substituiertes Phenyl, Phenoxy, Benzyloxy, Phenylthio und Benzylthio;
für gegebenenfalls einfach oder mehrfach, gleich oder verschieden durch Fluor, Chlor und/oder Brom substituiertes C₂-C₆-Alkenyl;
für C₃-C₈-Cycloalkyl, das gegebenenfalls einfach oder mehrfach, gleich oder verschieden substituiert ist durch
Fluor, Chlor, C₁-C₄-Alkyl, C₂-C₄-Alkenyl, sowie jeweils durch Fluor und/oder Chlor substituiertes C₁-C₂-Alkyl oder C₂-C₄-Alkenyl;
sowie für C₅-C₇-Cycloalkenyl, das gegebenenfalls einfach oder mehrfach, gleich oder verschieden substituiert ist durch
Fluor, Chlor, C₁-C₄-Alkyl sowie durch Fluor und/oder Chlor substituiertes C₁-C₂-Alkyl und
- B: steht bevorzugt für jeweils einfach bis vierfach, gleich oder verschieden substituiertes Phenyl, Benzyl, Pheneth-1-yl, Pheneth-2-yl, Phenoxymethyl, Phenylthiomethyl, Phenoxyeth-1-yl, Phenoxyeth-2-yl, Phenylthioeth-1-yl oder Styryl,
wobei jeweils als Phenylsubstituenten genannt seien
F, Cl, Br,
C₁-C₁₈-Alkyl,
C₁-C₆-Alkoxy-C₁-C₈-alkyl,
einfach bis sechsfach, gleich oder verschieden durch F und/oder Cl substituiertes C₁-C₈-Alkoxy,
einfach bis fünffach, gleich oder verschieden durch F und/oder Cl substituiertes C₁-C₂-Alkyl,
C₁-C₁₈-Alkoxy und -(OC₂H₄)₁₋₃-O-C₁-C₆-alkyl,
C₁-C₁₂-Alkylthio,
einfach bis sechsfach, gleich oder verschieden durch F und/oder Cl substituiertes C₁-C₈-Alkylthio,
3,4-Difluormethylendioxo,
3,4-Tetrafluorethylendioxo,
die Gruppierungen 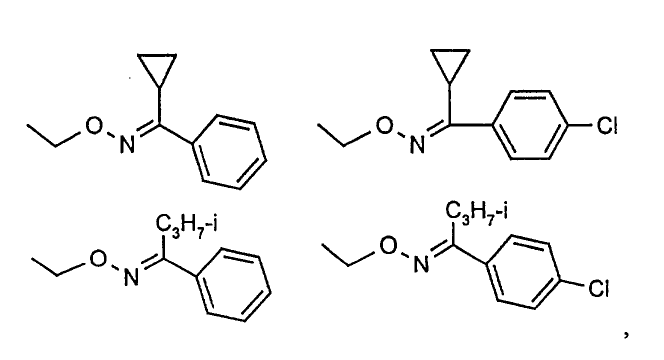 jeweils gegebenenfalls durch C₁-C₄-Alkyl, C₁-C₄-Alkoxy, Cyclohexyl oder Phenyl substituiertes Cyclohexyl und Cyclohexyloxy;
gegebenenfalls einfach oder zweifach, gleich oder verschieden durch F, Cl oder CF₃ substituiertes Pyridyloxy;
jeweils einfach bis dreifach, gleich oder verschieden durch C₁-C₁₂-Alkyl, F, Cl, Br, CF₃, C₁-C₄-Alkoxy, einfach bis sechsfach, gleich oder verschieden durch F und/oder Cl substituiertes C₁-C₄-Alkoxy, C₁-C₄-Alkoxy-C₁-C₄-alkyl, C₁-C₄-Alkoxy-ethylenoxy, C₁-C₄-Alkylthio, einfach bis sechsfach, gleich oder verschieden durch F und/oder Cl substituiertes C₁-C₄-Alkylthio substituiertes Phenoxy, Phenylthio, Benzyloxy und Benzylthio,
- D: steht bevorzugt für Wasserstoff oder Methyl;
- E: steht bevorzugt für Wasserstoff oder Methyl ; und
- G: steht bevorzugt für Wasserstoff oder Methyl.

Bevorzugte erfindungsgemäße Verbindungen sind auch Stoffgruppen der Formeln (Ia-1) bis (Ia-10): 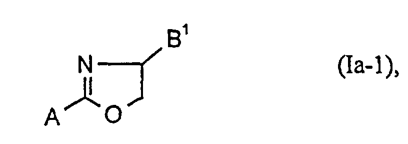 in welcher
- A: für die oben genannten allgemeinen und bevorzugten Bedeutungen steht und
- B¹: für einfach bis vierfach, gleich oder verschieden substituiertes Phenyl steht, wobei als Substituenten die oben unter (B) als bevorzugt genannten Phenylsubstituenten infrage kommen. 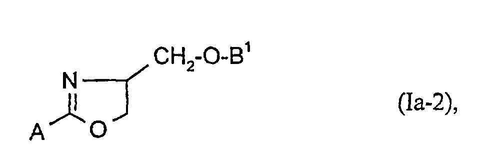 in welcher
- A: für die oben genannten allgemeinen und bevorzugten Bedeutungen steht und
- B¹: für einfach bis vierfach, gleich oder verschieden substituiertes Phenyl steht, wobei als Substituenten die oben unter (B) als bevorzugt genannten Phenylsubstituenten infrage kommen. 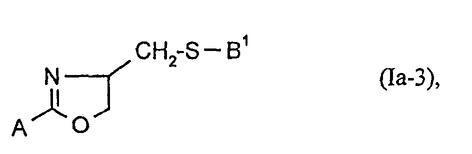 in welcher
- A: für die oben genannten allgemeinen und bevorzugten Bedeutungen steht und
- B¹: für einfach bis vierfach, gleich oder verschieden substituiertes Phenyl steht, wobei als Substituenten die oben unter (B) als bevorzugt genannten Phenylsubstituenten infrage kommen. 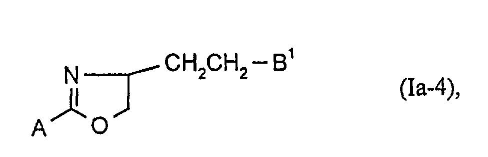 in welcher
- A: für die oben genannten allgemeinen und bevorzugten Bedeutungen steht und
- B¹: für einfach bis vierfach, gleich oder verschieden substituiertes Phenyl steht, wobei als Substituenten die oben unter (B) als bevorzugt genannten Phenylsubstituenten infrage kommen. 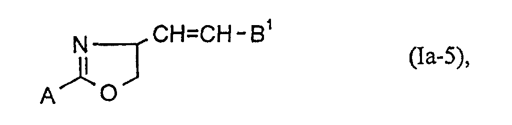 in welcher
- A: für die oben genannten allgemeinen und bevorzugten Bedeutungen steht und
- B¹: für einfach bis vierfach, gleich oder verschieden substituiertes Phenyl steht, wobei als Substituenten die oben unter (B) als bevorzugt genannten Phenylsubstituenten infrage kommen. 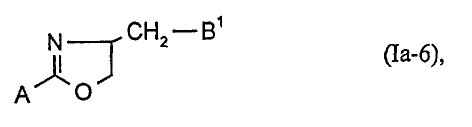 in welcher
- A: für die oben genannten allgemeinen und bevorzugten Bedeutungen steht und
- B¹: für einfach bis vierfach, gleich oder verschieden substituiertes Phenyl steht, wobei als Substituenten die oben unter (B) als bevorzugt genannten Phenylsubstituenten infrage kommen. 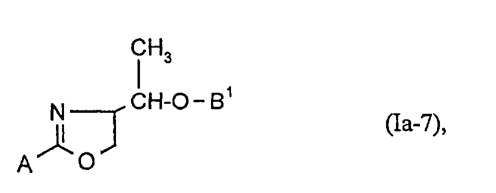 in welcher
- A: für die oben genannten allgemeinen und bevorzugten Bedeutungen steht und
- B¹: für einfach bis vierfach, gleich oder verschieden substituiertes Phenyl steht, wobei als Substituenten die oben unter (B) als bevorzugt genannten Phenylsubstituenten infrage kommen. 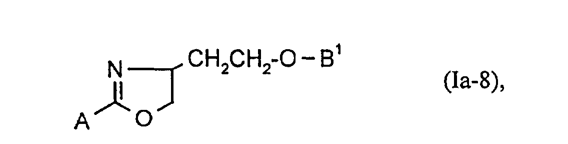 in welcher
- A: für die oben genannten allgemeinen und bevorzugten Bedeutungen steht und
- B¹: für einfach bis vierfach, gleich oder verschieden substituiertes Phenyl steht, wobei als Substituenten die oben unter (B) als bevorzugt genannten Phenylsubstituenten infrage kommen. 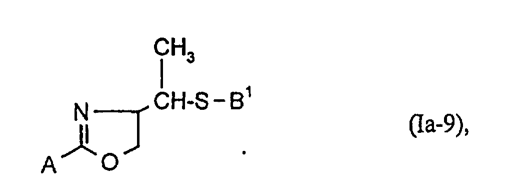 in welcher
- A: für die oben genannten allgemeinen und bevorzugten Bedeutungen steht und
- B¹: für einfach bis vierfach, gleich oder verschieden substituiertes Phenyl steht, wobei als Substituenten die oben unter (B) als bevorzugt genannten Phenylsubstituenten infrage kommen. 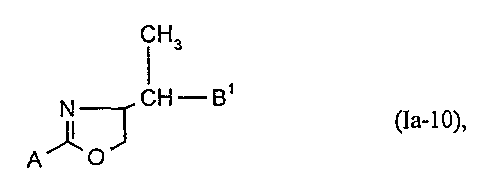 in welcher
- A: für die oben genannten allgemeinen und bevorzugten Bedeutungen steht und
- B¹: für einfach bis vierfach, gleich oder verschieden substituiertes Phenyl steht, wobei als Substituenten die oben unter (B) als bevorzugt genannten Phenylsubstituenten infrage kommen.

Weitere bevorzugte erfindungsgemäße Verbindungen sind Stoffgruppen der Formeln (Ia-1) bis (Ia-10), in denen A für gegebenenfalls einfach bis dreifach, gleich oder verschieden substituiertes tert.-Butyl steht, wobei als Substituenten die oben unter (A) als bevorzugt genannten Alkyl-Substituenten infrage kommen.

Ebenfalls bevorzugte erfindungsgemäße Verbindungen sind Stoffgruppen der Formeln (Ia-1) bis (Ia-10), in denen A für gegebenenfalls einfach bis fünffach, gleich oder verschieden substituiertes Cyclopropyl steht, wobei als Substituenten die oben unter (A) als bevorzugt genannten Cycloalkyl-Substituenten infrage kommen.

Außerdem bevorzugte erfindungsgemäße Verbindungen sind Stoffgruppen der Formeln (Ia-1) bis (Ia-10), in denen A für einfach bis dreifach, gleich oder verschieden durch Fluor und/oder Chlor substituiertes Vinyl steht.

Weiterhin bevorzugte erfindungsgemäße Verbindungen sind Stoffgruppen der Formeln (Ia-11) bis (Ia-20): 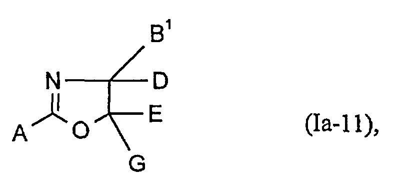 in welcher
- A: für die oben genannten allgemeinen und bevorzugten Bedeutungen steht;
- B¹: für einfach bis vierfach, gleich oder verschieden substituiertes Phenyl steht, wobei als Substituenten die oben unter (B) als bevorzugt genannten Phenylsubstituenten infrage kommen; und
- D, E und G: gleich oder verschieden sind und für Wasserstoff oder Methyl stehen, wobei jedoch mindestens ein Substituent für Methyl steht. 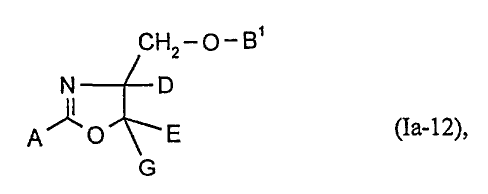 in welcher
- A: für die oben genannten allgemeinen und bevorzugten Bedeutungen steht;
- B¹: für einfach bis vierfach, gleich oder verschieden substituiertes Phenyl steht, wobei als Substituenten die oben unter (B) als bevorzugt genannten Phenylsubstituenten infrage kommen; und
- D, E und G: gleich oder verschieden sind und für Wasserstoff oder Methyl stehen, wobei jedoch mindestens ein Substituent für Methyl steht. 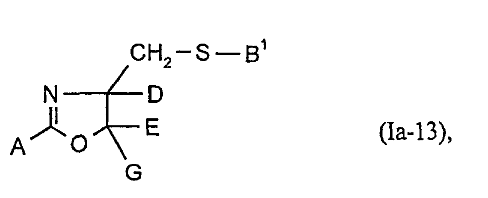 in welcher
- A: für die oben genannten allgemeinen und bevorzugten Bedeutungen steht;
- B¹: für einfach bis vierfach, gleich oder verschieden substituiertes Phenyl steht, wobei als Substituenten die oben unter (B) als bevorzugt genannten Phenylsubstituenten infrage kommen; und
- D, E und G: gleich oder verschieden sind und für Wasserstoff oder Methyl stehen, wobei jedoch mindestens ein Substituent für Methyl steht. 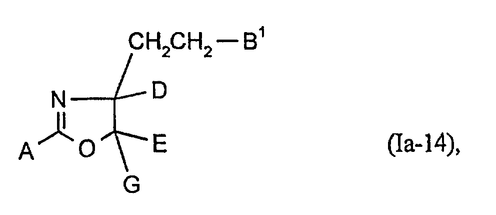 in welcher
- A: für die oben genannten allgemeinen und bevorzugten Bedeutungen steht;
- B¹: für einfach bis vierfach, gleich oder verschieden substituiertes Phenyl steht, wobei als Substituenten die oben unter (B) als bevorzugt genannten Phenylsubstituenten infrage kommen; und
- D, E und G: gleich oder verschieden sind und für Wasserstoff oder Methyl stehen, wobei jedoch mindestens ein Substituent für Methyl steht. 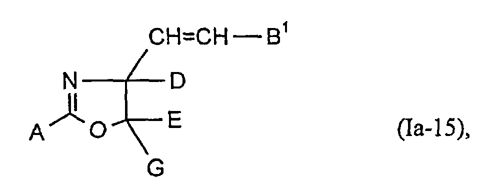 in welcher
- A: für die oben genannten allgemeinen und bevorzugten Bedeutungen steht;
- B¹: für einfach bis vierfach, gleich oder verschieden substituiertes Phenyl steht, wobei als Substituenten die oben unter (B) als bevorzugt genannten Phenylsubstituenten infrage kommen; und
- D, E und G: gleich oder verschieden sind und für Wasserstoff oder Methyl stehen, wobei jedoch mindestens ein Substituent für Methyl steht. 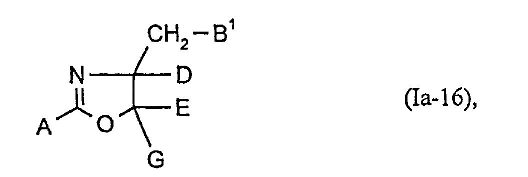 in welcher
- A: für die oben genannten allgemeinen und bevorzugten Bedeutungen steht;
- B¹: für einfach bis vierfach, gleich oder verschieden substituiertes Phenyl steht, wobei als Substituenten die oben unter (B) als bevorzugt genannten Phenylsubstituenten infrage kommen; und
- D, E und G: gleich oder verschieden sind und für Wasserstoff oder Methyl stehen, wobei jedoch mindestens ein Substituent für Methyl steht. 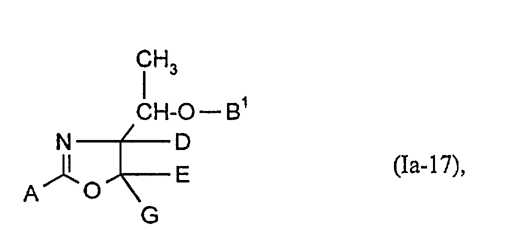 in welcher
- A: für die oben genannten allgemeinen und bevorzugten Bedeutungen steht;
- B¹: für einfach bis vierfach, gleich oder verschieden substituiertes Phenyl steht, wobei als Substituenten die oben unter (B) als bevorzugt genannten Phenylsubstituenten infrage kommen; und
- D, E und G: gleich oder verschieden sind und für Wasserstoff oder Methyl stehen, wobei jedoch mindestens ein Substituent für Methyl steht. 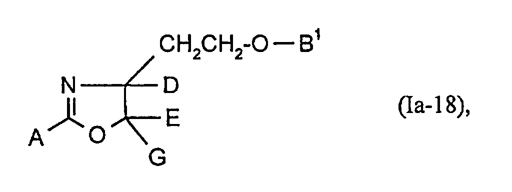 in welcher
- A: für die oben genannten allgemeinen und bevorzugten und Bedeutungen steht;
- B¹: für einfach bis vierfach, gleich oder verschieden substituiertes Phenyl steht, wobei als Substituenten die oben unter (B) als bevorzugt genannten Phenylsubstituenten infrage kommen; und
- D, E und G: gleich oder verschieden sind und für Wasserstoff oder Methyl stehen, wobei jedoch mindestens ein Substituent für Methyl steht. 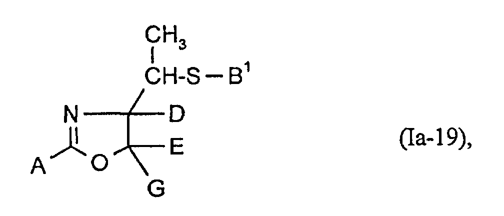 in welcher
- A: für die oben genannten allgemeinen und bevorzugten Bedeutungen steht;
- B¹: für einfach bis vierfach, gleich oder verschieden substituiertes Phenyl steht, wobei als Substituenten die oben unter (B) als bevorzugt genannten Phenylsubstituenten infrage kommen; und
- D, E und G: gleich oder verschieden sind und für Wasserstoff oder Methyl stehen, wobei jedoch mindestens ein Substituent für Methyl steht. 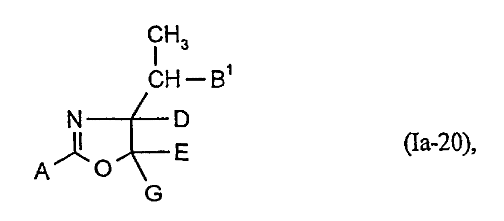 in welcher
- A: für die oben genannten allgemeinen und bevorzugten Bedeutungen steht;
- B¹: für einfach bis vierfach, gleich oder verschieden substituiertes Phenyl steht, wobei als Substituenten die oben unter (B) als bevorzugt genannten Phenylsubstituenten infrage kommen; und
- D, E und G: gleich oder verschieden sind und für Wasserstoff oder Methyl stehen, wobei jedoch mindestens ein Substituent für Methyl steht.

Weitere bevorzugte erfindungsgemäße Verbindungen sind Stoffgruppen der Formeln (Ia-11) bis (Ia-20), in denen A für gegebenenfalls einfach bis dreifach, gleich oder verschieden substituiertes tert.-Butyl steht, wobei als Substituenten die oben unter (A) als bevorzugt genannten Alkyl-Substituenten infrage kommen.

Ebenfalls bevorzugte erfindungsgemäße Verbindungen sind Stoffgruppen der Formeln (Ia-11) bis (Ia-20), in denen A für gegebenenfalls einfach bis fünffach, gleich oder verschieden substituiertes Cyclopropyl steht, wobei als Substituenten die oben unter (A) als bevorzugt genannten Cycloalkyl-Substituenten infrage kommen.

Außerdem bevorzugte erfindungsgemäße Verbindungen sind Stoffgruppen der Formeln (Ia-11) bis (Ia-20), in denen A für einfach bis dreifach, gleich oder verschieden durch Fluor und/oder Chlor substituiertes Vinyl steht.

Die oben bei der Definition der erfindungsgemäßen Verbindungen genannten Kohlenwasserstoffreste, wie Alkyl sind - auch in Verbindung mit Heteroatomen, wie Alkoxy - soweit möglich, jeweils geradkettig oder verzweigt.

Beispielhaft seien für den Substituenten A folgende Reste genannt:

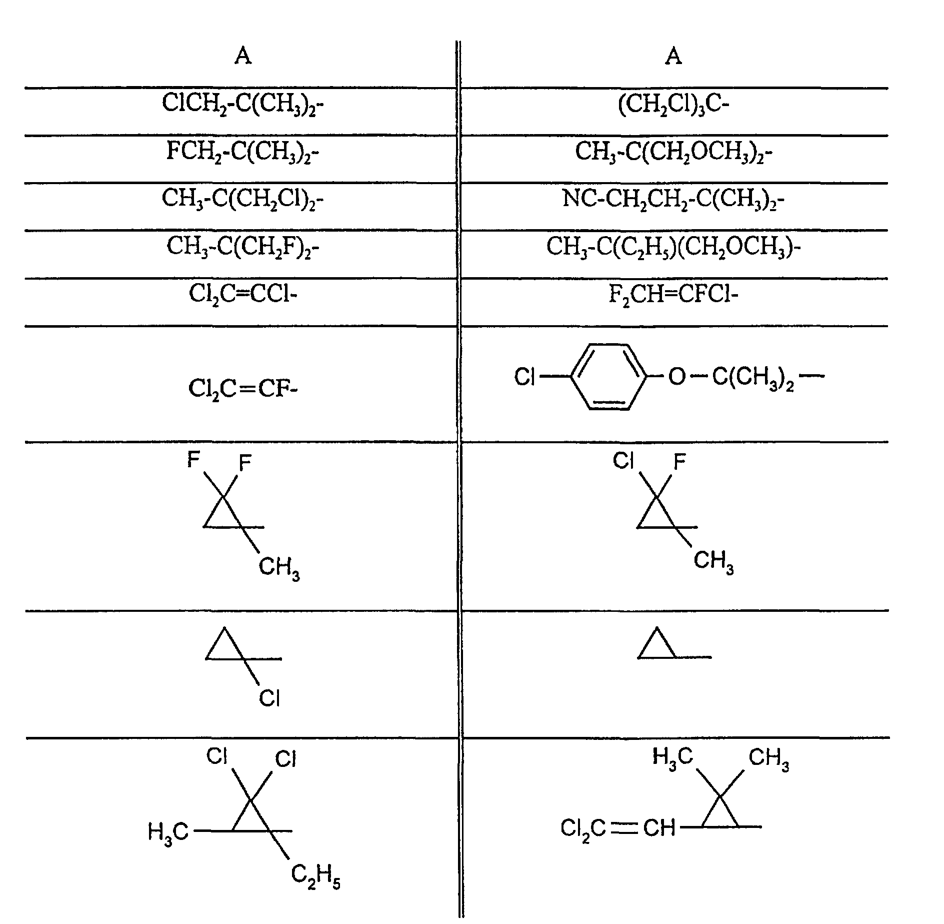

Beispielhaft seien für den Substituenten B folgende Reste genannt: 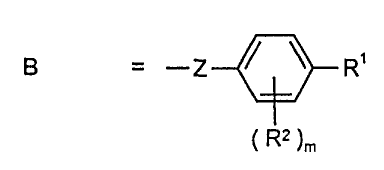 wobei
- Z =: direkte Bindung, -CH₂-, -CH(CH₃)-, -CH₂CH₂-, -CH=CH-, -CH₂O-, -CH₂S-, -CH(CH₃)S-, -CH(CH₃)O-, -CH₂CH₂O-
- R¹ =: gemäß Tabelle 1 und
- (R²)ₘ =: gemäß Tabelle 2.

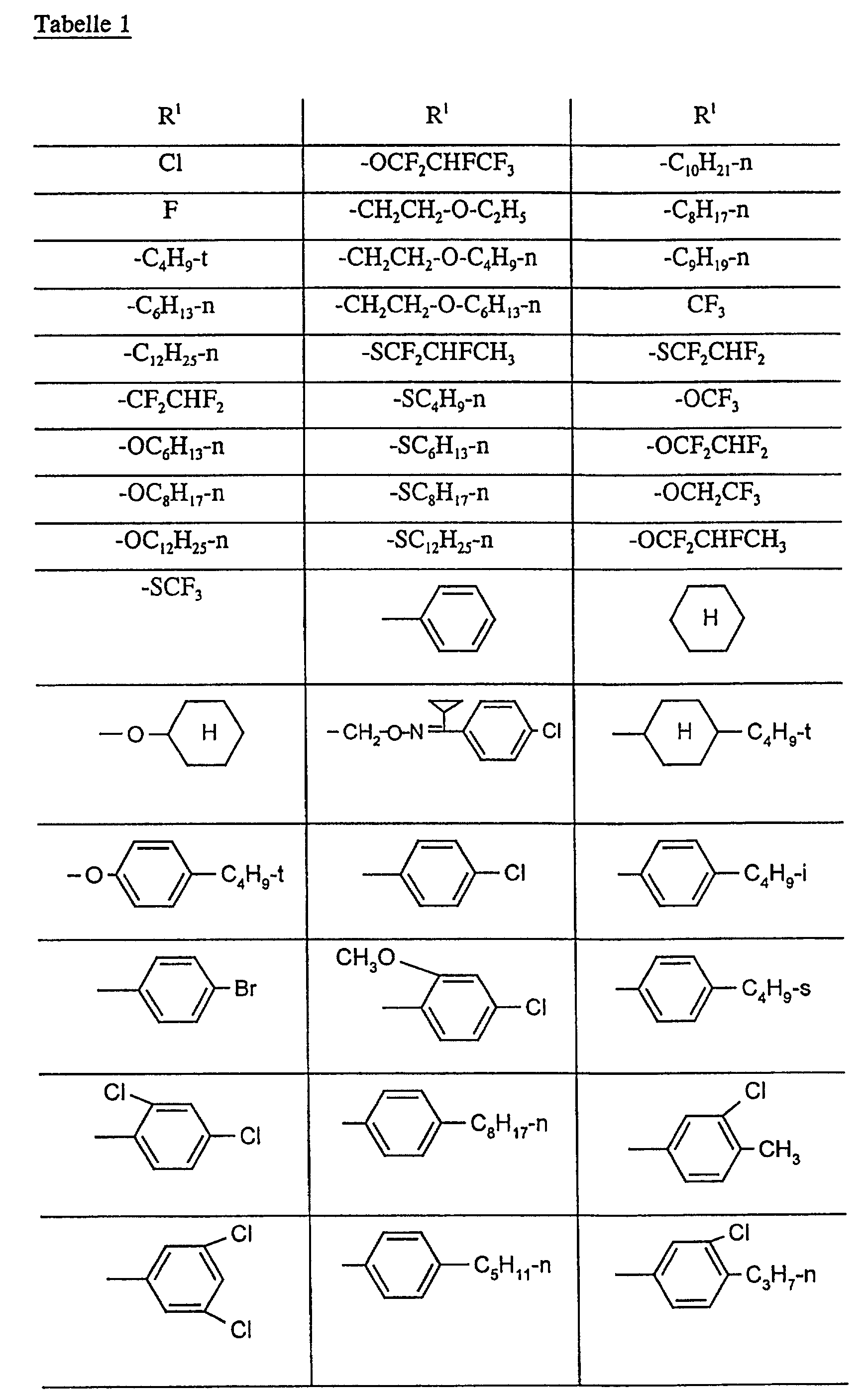

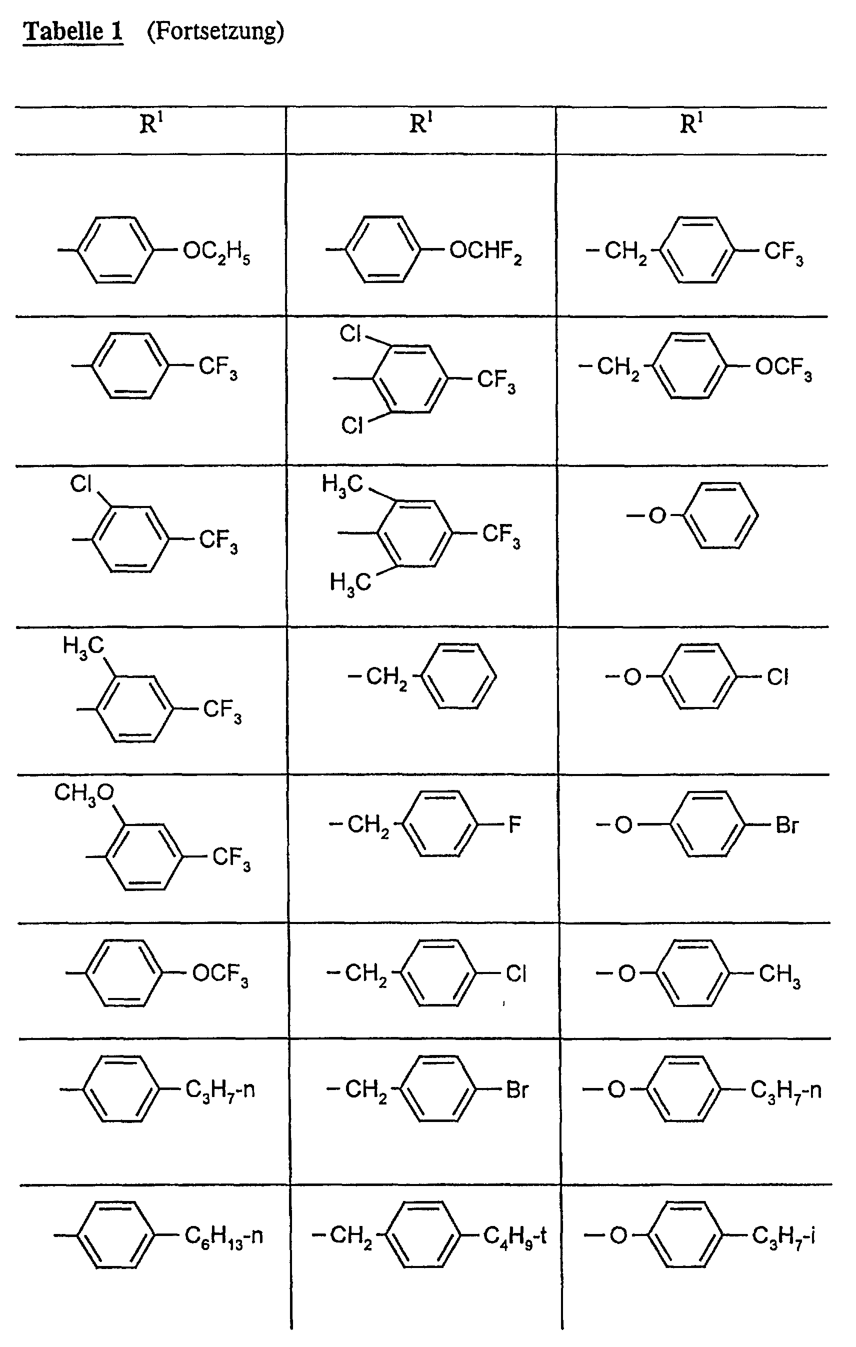

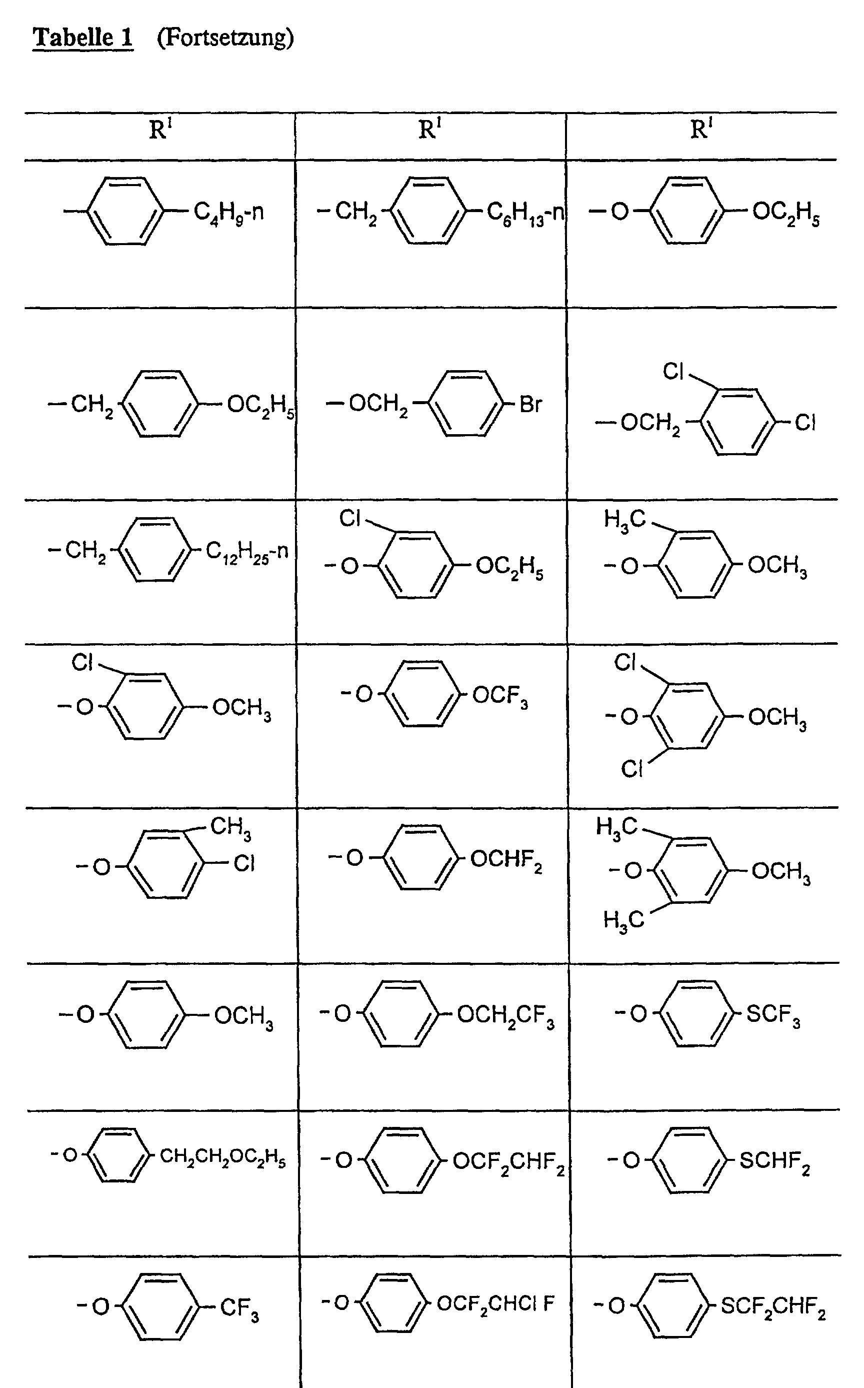

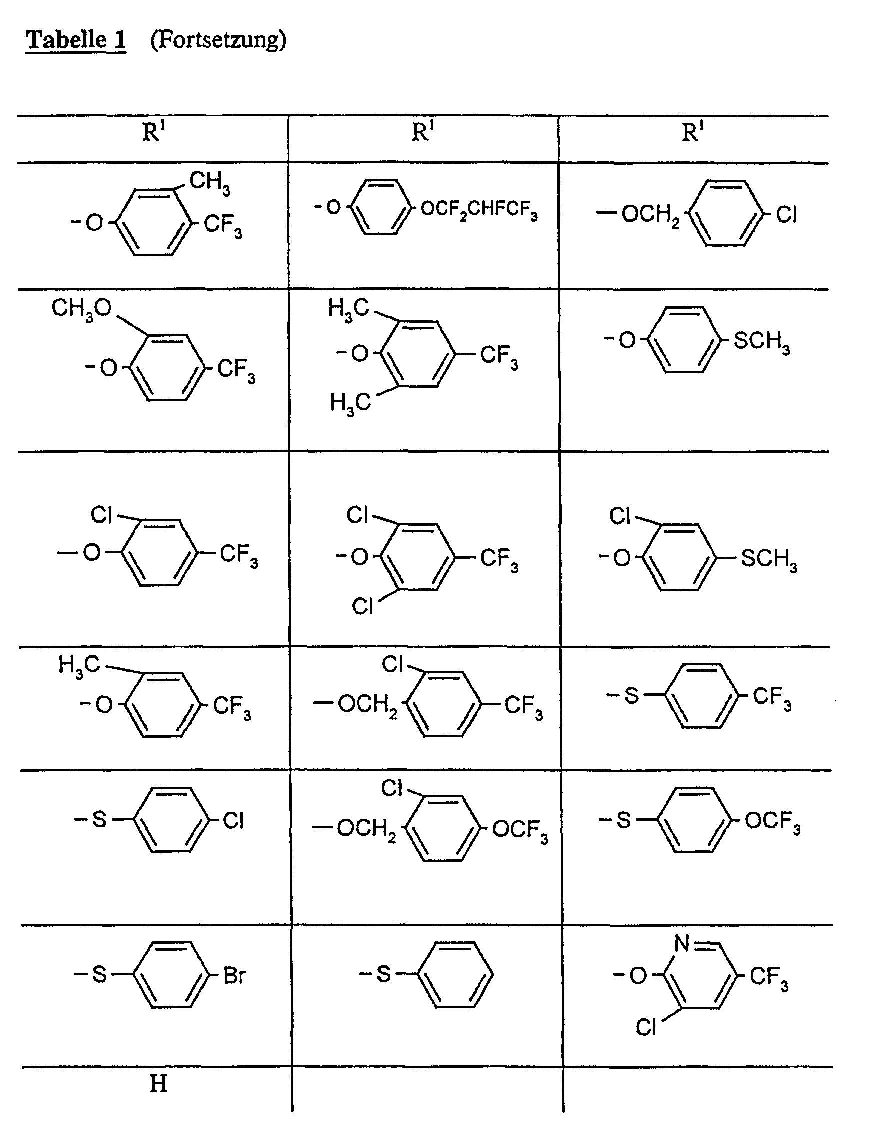

**Tabelle 2**

| (R²)ₘ | (R²)ₘ |
|---|---|
| H | 2-CH₃ |
| 2-Cl | 2-OCH₃ |
| 2-F | 2-OC₂H₅ |
| 3-Cl | 3-CH₃ |
| 2,6-Cl₂ | 3,5-(CH₃)₂ |
| 3,5-Cl₂ | 3-OC₆H₅ |
| 3,5-F₂ | |
| 2,5-Cl₂ | gemeinsam mit R¹ für |
| 3,5-Cl₂;2-F | |
| 2,3-F₂ | 3,4-OCF₂O-und |
| 2,5-F₂ | 3,4-OCF₂CF₂O- |

wobei R¹ und R² nicht gleichzeitig für Wasserstoff stehen dürfen.

Im einzelnen seien folgende Beispiele für die erfindungsgemäßen Verbindungen der Formel (Ia) genannt:

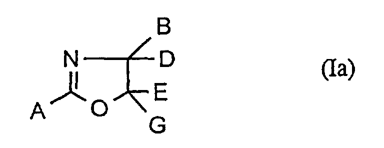

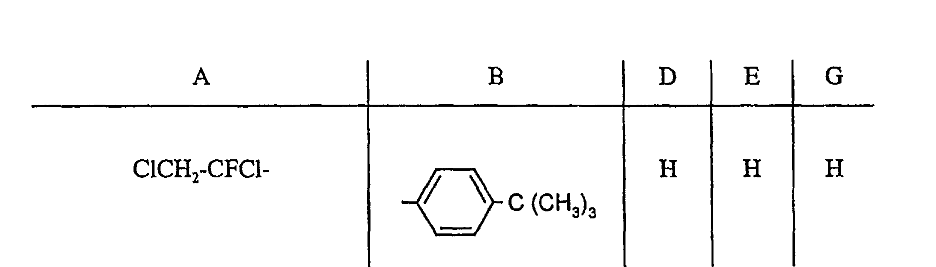

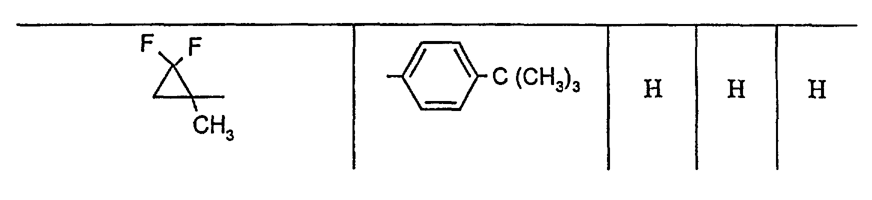

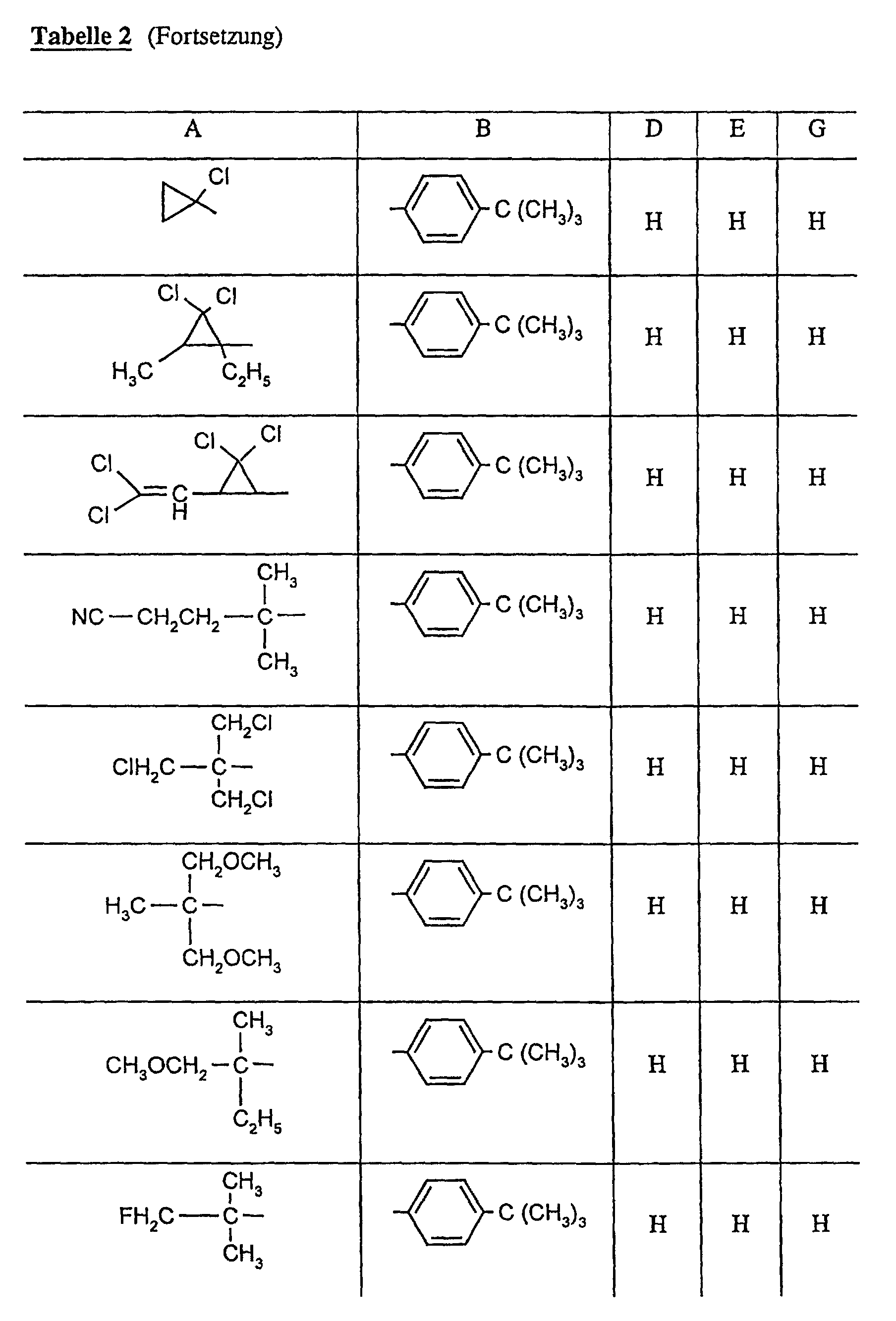

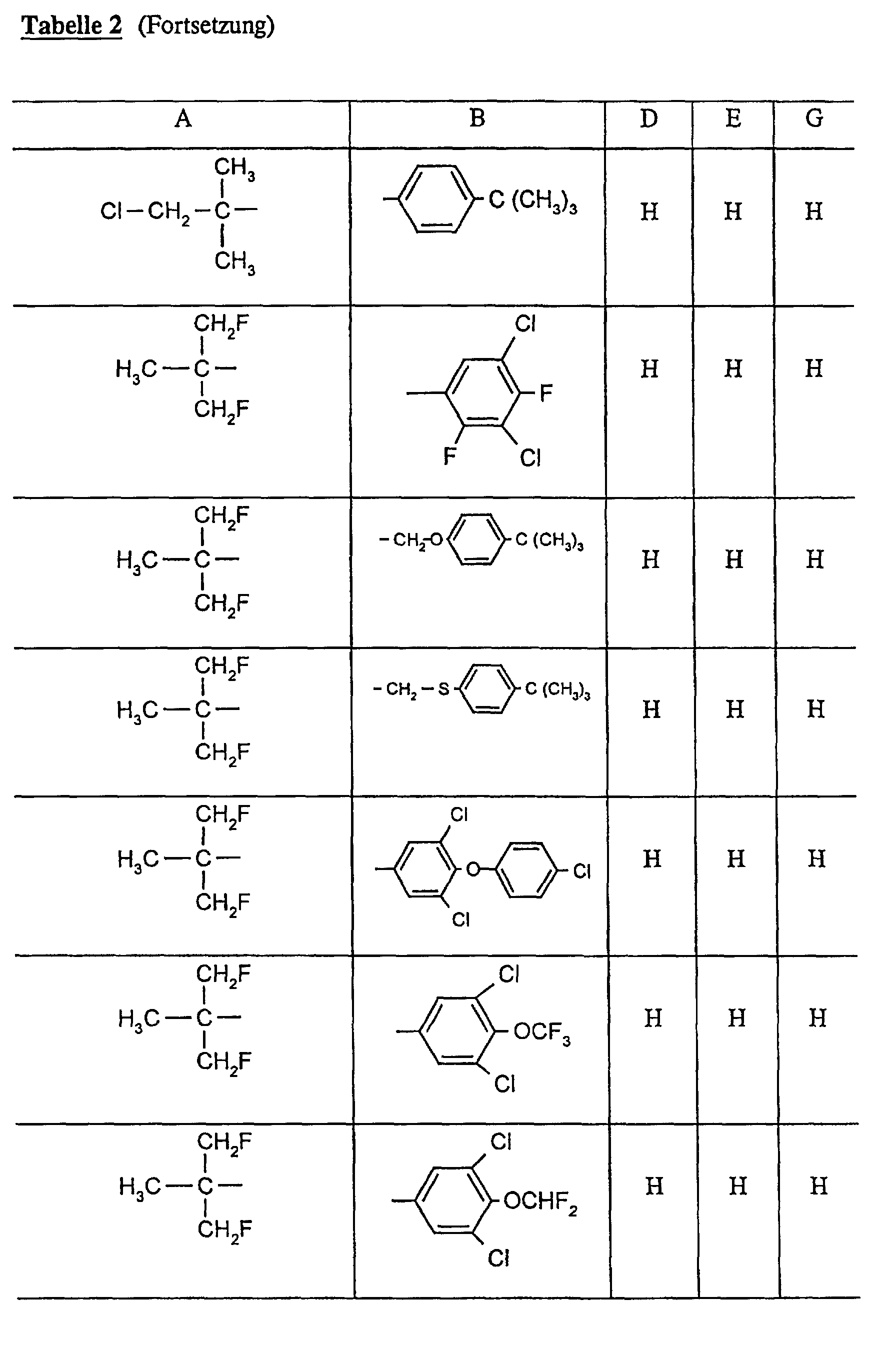

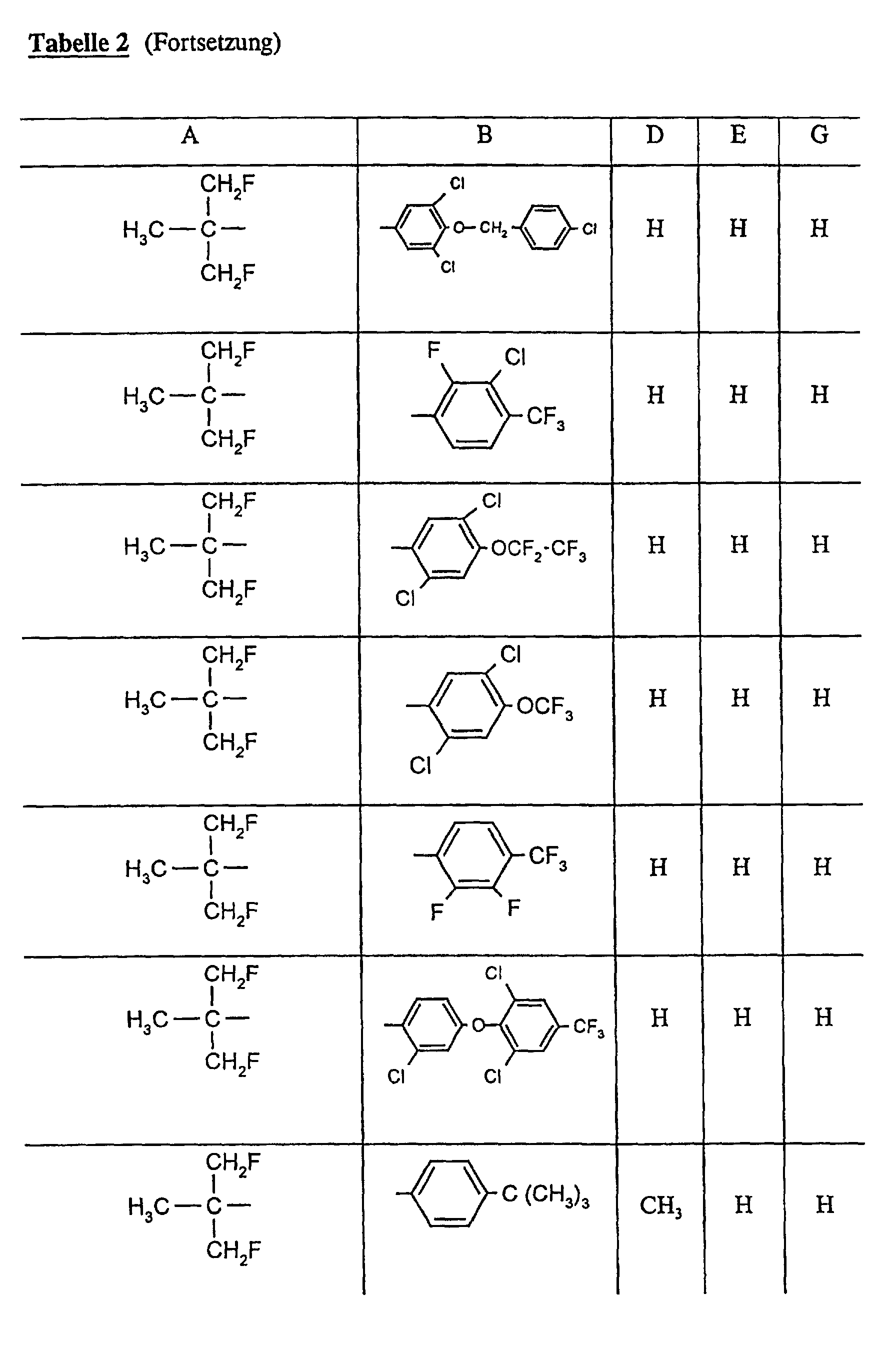

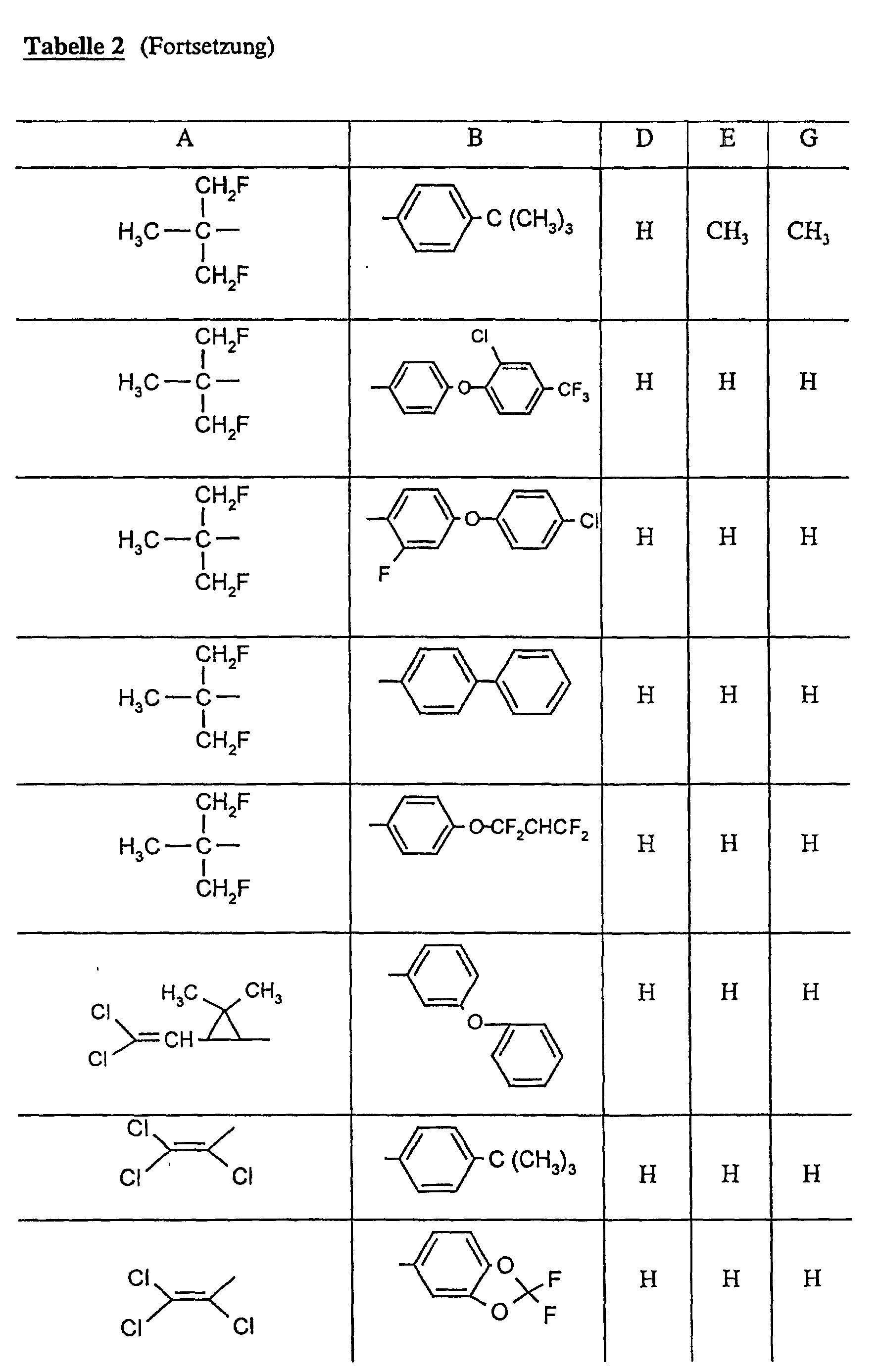

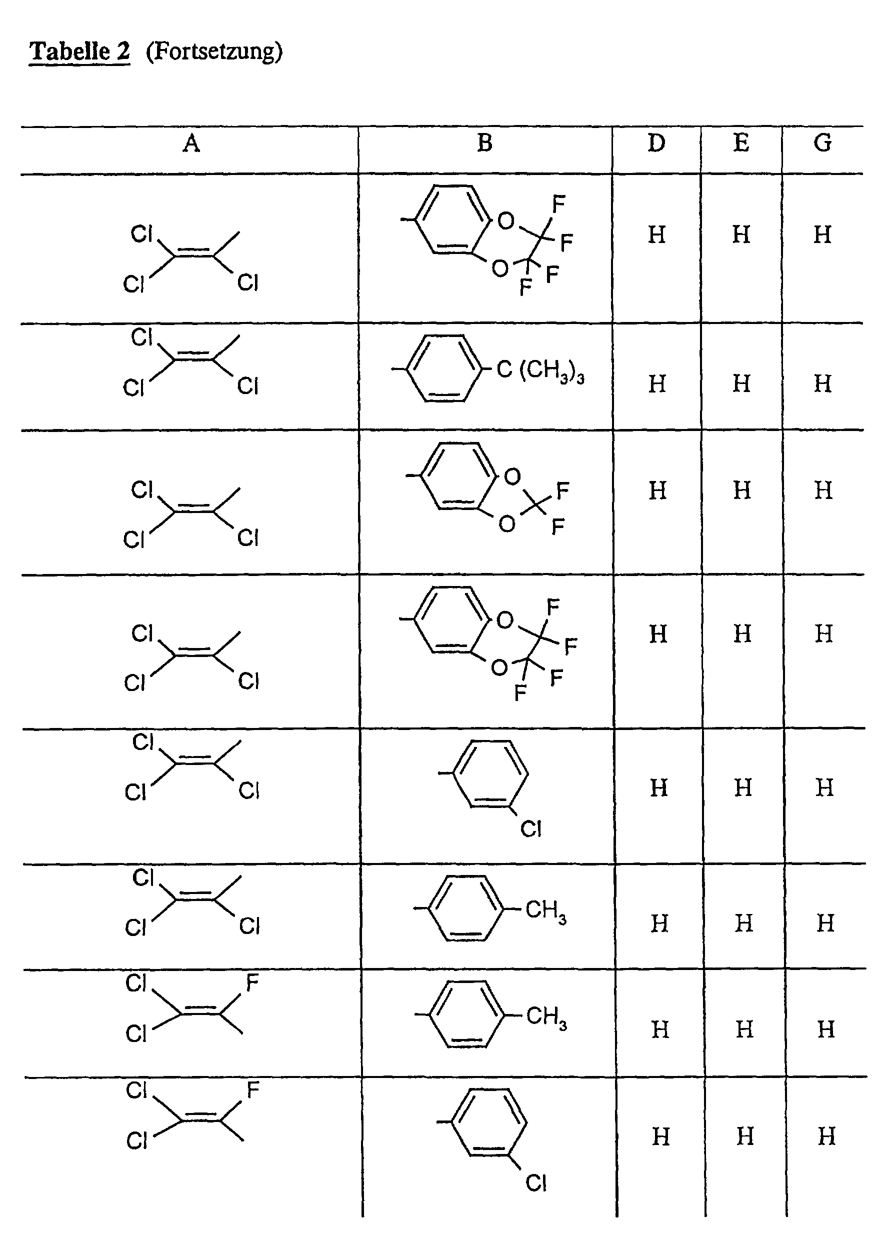

Verwendet man zur Durchführung des erfindungsgemäßen Verfahrens (a) beispielsweise 2-Amino-2-(4-t-butylphenyl)-1-ethanol und 2,2-Bis(chlormethyl)-propionsäure als Ausgangsstoffe und Polyphosphorsäure (PPS) als Wasser entziehendes Mittel, so kann der Reaktionsablauf durch das folgende Formelschema skizziert werden:

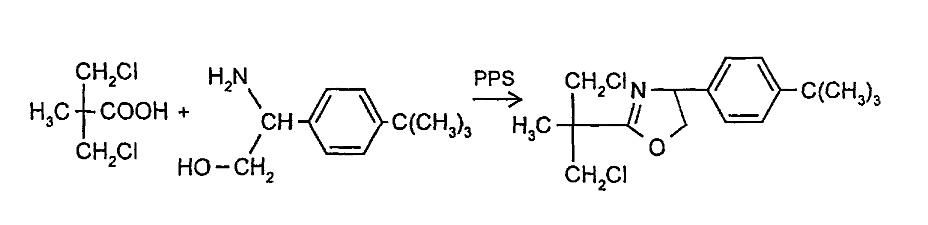

Verwendet man zur Durchführung des erfindungsgemäßen Verfahrens (b) beispielsweise N-[2-Hydroxy-1-(4-t-butylphenyl)-ethyl]-2,2-bis(chlormethyl)-propionsäureamid als Ausgangsverbindung und Polyphosphorsäure (PPS) als Wasser entziehendes Mittel, so kann der Reaktionsablauf durch das folgende Formelschema skizziert werden:

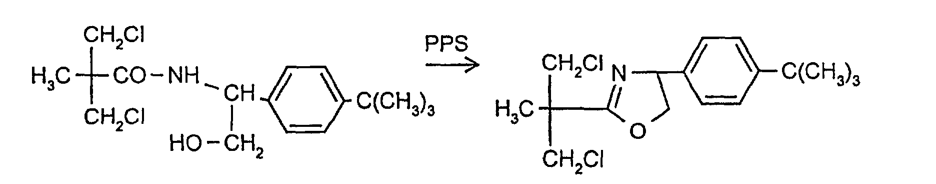

Verwendet man zur Durchführung des erfindungsgemäßen Verfahrens (c) beispielsweise N-[2-Chlor-1-(4-t-butylphenyl)-ethyl]-2,2-ks(chlormethyl)-propionsäureamid als Ausgangsverbindung und Triethylamin als Base, so kann der Reaktionsablauf durch das folgende Formelschema skizziert werden:

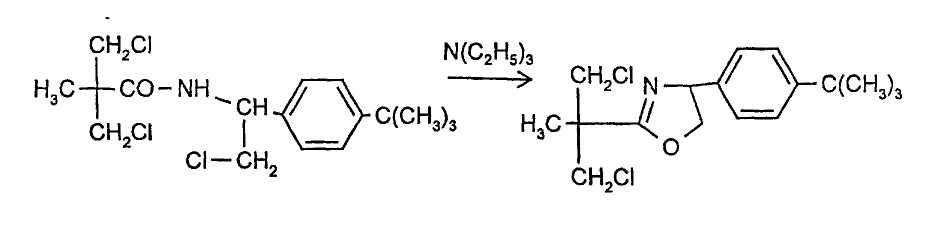

Die beim erfindungsgemäßen Verfahren (a) zur Herstellung der Verbindungen der Formel (Ia) als Ausgangsstoffe zu verwendenden Aminoalkohole sind durch die Formel (II) allgemein definiert. In der Formel (II) haben B, D, E und G vorzugsweise diejenigen Bedeutungen, die bereits oben im Zusammenhang mit der Beschreibung der Verbindungen der Formel (Ia) als bevorzugt für B, D, E und G angegeben wurden.

Die Ausgangsstoffe der Formel (II) sind bekannt und/oder können nach an sich bekannten Verfahren durch Reduktion der entsprechenden Aminosäuren hergestellt werden (vgl. Heterocycles 9 (1978), 1277-1285; J. Org. Chem. 43 (1978), 2539-2541; Liebigs Ann. Chem. 1980, 122-139; Tetrahedron Lett. 26 (1985), 4971-4974).

Die beim erfindungsgemäßen Verfahren (a) zur Herstellung der Verbindungen der Formel (Ia) weiter als Ausgangsstoffe zu verwendenden Carbonsäuren sind durch die Formel (III) allgemein definiert. In der Formel (III) hat A vorzugsweise diejenige Bedeutung, die bereits oben im Zusammenhang mit der Beschreibung der Verbindungen der Formel (Ia) als bevorzugt für A angegeben wurde.

Die Ausgangsstoffe der Formel (III) sind bekannte organischen Synthesechemikalien, bzw. in bekannter Art und Weise erhältlich.

Die erfindungsgemäßen Verfahren (a) und (b) werden unter Verwendung eines wasser-entziehenden Mittels durchgeführt. Es können die in der organischen Chemie üblichen wasser-entziehenden Mittel eingesetzt werden. Vorzugsweise verwendbar sind Schwefelsäure, Polyphosphorsäure (PPS), Phosphor(V)-oxid, Dicyclohexylcarbodiimid (DCC), Phosphor(V)-sulfid und das System Triphenylphosphin/Triethylamin/Tetrachlormethan.

Als Verdünnungsmittel zur Durchführung der erfindungsgemäßen Verfahren (a) bis (c) kommen die üblichen organischen Lösungsmittel in Betracht. Vorzugsweise verwendbar sind aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlormethan; Ether, wie Diethylether, Diisopropylether, Dioxan, Tetrahydrofuran oder Ethylenglykoldimethyl- oder -diethylether; Ketone, wie Aceton, Butanon oder Methylisobutyl-keton; Nitrile, wie Acetonitril, Propionitril oder Benzonitril; Amide, wie N,N-Dimethylformamid und N,N-Dimethylacetamid, N-Methyl-pyrrolidon oder Hexamethylphosphorsäuretriamid; Ester wie Essigsäuremethylester oder Essigsäureethylester, sowie Sulfoxide, wie Dimethylsulfoxid, gegebenenfalls auch Alkohole wie Methanol oder Ethanol.

Die Reaktionstemperaturen können bei der Durchführung des erfindungsgemäßen Verfahrens (a) in einem größeren Bereich variiert werden. Im allgemeinen arbeitet man bei Temperaturen zwischen 0°C und 150°C, vorzugsweise bei Temperaturen zwischen 10°C und 100°C.

Das erfindungsgemäße Verfahren (a) wird im allgemeinen unter Normaldruck durchgeführt. Es ist jedoch auch möglich, unter erhöhtem oder vermindertem Druck - im allgemeinen zwischen 0,1 bar und 10 bar - zu arbeiten.

Zur Durchführung des erfindungsgemäßen Verfahrens (a) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines wasser-entziehenden Mittels durchgefiihrt, und das Reaktionsgemisch wird mehrere Stunden bei der jeweils erforderlichen Temperatur gerührt. Die Aufarbeitung erfolgt nach üblichen Methoden.

In einer besonderen Ausführungsform des erfindungsgemäßen Verfahrens (a) können statt der Carbonsäuren der Formel (III) auch entsprechende Nitrile eingesetzt werden, wobei dann vorzugsweise an Stelle eines wasser-entziehenden Mittels ein Katalysator, wie z.B. Zink-(II)-chlorid verwendet wird.

Die bei dem erfindungsgemäßen Verfahren (b) zur Herstellung der Verbindungen der Formel (Ia) als Ausgangsstoffe zu verwendenden Amidalkohole sind durch die Formel (IV) allgemein definiert. In der Formel (IV) haben A, B, D, E und G vorzugsweise diejenigen Bedeutungen, die bereits oben im Zusammenhang mit der Beschreibung der Verbindungen der Formel (Ia) als bevorzugt für A, B, D, E und G angegeben wurden.

Die Ausgangsstoffe der Formel (IV) sind bekannt und/oder können nach an sich bekannten Verfahren hergestellt werden.

Man erhält die Amidalkohole der Formel (IV) beispielsweise, wenn man von den Carbonsäuren der Formel (III) abgeleitete Säurechloride mit Aminoalkoholen der Formel (II) in Gegenwart eines Säurebindemittels, wie z.B. Triethylamin, Pyridin, Kaliumcarbonat, Natriumhydroxid oder Kalium-t-butylat, und in Gegenwart eines Verdünnungsmittels, wie z.B. Toluol, Chlorbenzol, Aceton oder Acetonitril, bei Temperaturen zwischen 0°C und 100°C umsetzt (vgl. die Herstellungsbeispiele).

Die von den Carbonsäuren der Formel (III) abgeleiteten Säurechloride sind weitgehend bekannt und/oder können nach an sich bekannten Verfahren, beispielsweise durch Umsetzung der Carbonsäuren der Formel (III) mit einem Halogenierungsmittel wie Thionylchlorid, gegebenenfalls in Gegenwart eines Verdünnungsmittels hergestellt werden.

Noch nicht bekannt ist α-Fluor-β,β-dichlor-acrylsäurechlorid der Formel (VI)

Cl₂C = CF - COCl (VI).

Es wird erhalten, indem man 2-Fluor-1,1,3,3,3-Pentachlorpropen gegebenenfalls in Gegenwart eines Katalysators hydrolysiert. Als Katalysatoren kommen Lewis-Säuren, anorganische Säuren und deren saure Salze, wie z.B. FeCl₃, BF₃, H₂SO₄, HCl, KHSO₄, NaHSO₄ u.a. in Frage (vgl. auch die Herstellungsbeispiele).

In Abhängigkeit von den Reaktionsbedingungen (wie beispielsweise der Verweilzeit des Säurechlorids in der Reaktionsmischung) und von der zugesetzten Wassermenge wird das primär gebildete α-Fluor-β,β-dichlor-acrylsäurechlorid gegebenenfalls teilweise zur entsprechenden Acrylsäure hydrolysiert, welche anschließend beispielsweise durch Umsetzung mit Thionylchlorid wieder in das Säurechlorid überführt werden kann.

Die Reaktionstemperaturen können bei der Durchführung des erfindungsgemäßen Verfahrens (b) in einem größeren Bereich variiert werden. Im allgemeinen arbeitet man bei Temperaturen zwischen -20°C und +150°C, vorzugsweise bei Temperaturen zwischen 0°C und 100°C.

Das erfindungsgemäße Verfahren (b) wird im allgemeinen unter Normaldruck durchgeführt. Es ist jedoch auch möglich, unter erhöhtem oder vermindertem Druck - im allgemeinen zwischen 0,1 bar und 10 bar - zu arbeiten.

Zur Durchführung des erfindungsgemäßen Verfahrens (b) zur Herstellung der Verbindungen der Formel (Ia) setzt man pro Mol an Amidalkohol der Formel (IV) im allgemeinen 1 bis 20 Mol, vorzugsweise 1 bis 5 Mol an wasser-entziehendem Mittel ein.

In einer bevorzugten Ausführungsform des erfindungsgemäßen Verfahrens (b) wird der Amidalkohol der Formel (IV) in einem Verdünnungsmittel vorgelegt und das wasserentziehende Mittel wird dann eindosiert. Das Reaktionsgemisch wird bei der erforderlichen Temperatur bis zum Ende der Umsetzung gerührt und anschließend auf übliche Weise aufgearbeitet.

Die beim erfindungsgemäßen Verfahren (c) zur Herstellung der Verbindungen der Formel (Ia) als Ausgangsstoffe zu verwendenden Amid-Derivate sind durch die Formel (V) allgemein definiert. In der Formel (V) haben A, B, D, E und G vorzugsweise diejenigen Bedeutungen, die bereits oben im Zusammenhang mit der Beschreibung der Verbindungen der Formel (Ia) als bevorzugt für A, B, D, E und G angegeben wurden; X steht vorzugsweise für Fluor, Chlor, Brom, Iod, C₁-C₄-Alkyl-sulfonyloxy, Phenylsulfonyloxy oder Tolylsulfonyloxy, insbesondere für Chlor, Brom, Methylsulfonyloxy oder Tolylsulfonyloxy.

Die Ausgangsstoffe der Formel (V) sind bekannt und/oder können nach an sich bekannten Verfahren hergestellt werden.

Man erhält die Amid-Derivate der Formel (V), wenn man entsprechende Amidalkohole der Formel (IV) mit Chlorierungsmitteln, wie z.B. Thionylchlorid oder Phosphor(V)chlorid, bzw. mit Sulfonylierungsmitteln, wie z.B. Methansulfonsäurechlorid oder p-Toluolsulfonsäurechlorid auf übliche Weise gegebenenfalls in Gegenwart einer Base und gegebenenfalls in Gegenwart eines Verdünnungsmittels umsetzt.

Das erfindungsgemäße Verfahren (c) wird in Gegenwart einer Base durchgeführt. Es kommen hierbei alle üblichen anorganischen oder organischen Basen in Betracht. Vorzugsweise verwendbar sind Erdalkali- oder Alkalimetallhydride, -hydroxide, -arnide, -alkoholate, -acetate, -carbonate oder -hydrogencarbonate, wie beispielsweise Natriumhydrid, Natriumamid, Natriummethylat, Natriumethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat sowie tertiäre Amine, wie Trimethylamin, Triethylamin, Tributylamin, N,N-Dimethylanilin, Pyridin, N-Methylpiperidin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (c) in einem größeren Bereich variiert werden. Im allgemeinen arbeitet man bei Temperaturen zwischen -20°C und +150°C, vorzugsweise bei Temperaturen zwischen 0°C und 100°C.

Das erfindungsgemäße Verfahren (c) wird im allgemeinen unter Normaldruck durchgeführt. Es ist jedoch auch möglich, unter erhöhtem oder vermindertem Druck - im allgemeinen zwischen 0,1 bar und 10 bar - zu arbeiten.

Zur Durchführung des erfindungsgemäßen Verfahrens (c) zur Herstellung der Verbindungen der Formel (Ia) setzt man pro Mol an Amid-Derivat der Formel (V) im allgemeinen 1 bis 3 Mol, vorzugsweise 1,0 bis 1,5 Mol einer Base ein.

In einer bevorzugten Ausführungsform des erfindungsgemäßen Verfahrens (c) werden das Amid-Derivat der Formel (V) und eine Base in einem geeigneten Verdünnungsmittel vermischt; das Gemisch wird bei der erforderlichen Temperatur bis zum Ende der Umsetzung gerührt und anschließend auf übliche Weise aufgearbeitet.

Die Wirkstoffe eignen sich zur Bekämpfung von tierischen Schädlingen, vorzugsweise Arthropoden und Nematoden, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:
Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofinannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Acanthoscelides obtectus, Bruchidius obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..

Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Zu den pflanzenparasitären Nematoden gehören Pratylenchus spp., Radopholus similis, Ditylenchus dipsaci, Tylenchulus semipenetrans, Heterodera spp., Meloidogyne spp., Aphelenchoides spp., Longidorus spp., Xiphinema spp., Trichodorus spp..

Die erfindungsgemäßen Verbindungen zeigen in entsprechenden Aufwandmengen auch eine fungizide Wirkung.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgae, wie Halogenkohlenwasserstoffe sowie Butan, Propan, Stickstoff und Kohlendioxid; als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in ihren handelsüblichen Formulierungen sowie in den aus den Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a..

Genannt seien die folgenden Verbindungen:
Acrinathrin, Alphamethrin, Betacyfluthrin, Bifenthrin, Brofenprox, Cis-Resmethrin, Clocythrin, Cycloprothrin, Cyfluthrin, Cyhalothrin, Cypermethrin, Deltamethrin, Esfenvalerate, Etofenprox, Fenpropathrin, Fenvalerate, Flucythrinate, Fluvalinate, Lambda-Cyhalothrin, Permethrin, Pyresmethrin, Pyrethrum, Silafluofen, Tralomethrin, Zetamethrin,
Alanycarb, Bendiocarb, Benfuracarb, Bufencarb, Butocarboxim, Carbaryl, Cartap, Ethiofencarb, Fenobucarb, Fenoxycarb, Isoprocarb, Methiocarb, Methomyl, Metolcarb, Oxamyl, Pirimicarb, Promecarb, Propoxur, Terbam, Thiodicarb, Thiofanox, Trimethacarb, XMC, Xylylcarb,
Acephate, Azinphos A, Azinphos M, Bromophos A, Cadusafos, Carbophenothion, Chlorfenvinphos, Chlormephos, Chlorpyrifos, Chlorpyrifos M, Cyanophos. Demeton M, Demeton-S-methyl, Demeton S, Diazinon, Dichlorvos, Dicliphos, Dichlorfenthion, Dicrotophos, Dimethoate, Dimethylvinphos, Dioxathion, Disulfoton, Edifenphos, Ethion, Etrimphos, Fenitrothion, Fenthion, Fonophos, Formothion, Heptenophos, Iprobenfos, Isazophos, Isoxathion, Phorate, Malathion, Mecarbam, Mervinphos, Mesulfenphos, Methacrifos, Methamidophos, Naled, Omethoate, Oxydemeton M, Oxydeprofos, Parathion A, Parathion M, Phenthoate, Phorate, Phosalone, Phosmet, Phosphamdon, Phoxim, Pirimiphos A, Pirimiphos M, Propaphos, Prothiophos, Prothoate, Pyraclophos, Pyridaphenthion, Quinalphos, Salithion, Sebufos, Sulfotep, Sulprofos, Tetrachlorvinphos, Temephos, Thiomethon, Thionazin, Trichlorfon, Triazophos, Vamidothion,
Buprofezin, Chlorfluazuron, Diflubenzuron, Flucycloxuron, Flufenoxuron, Hexaflumuron, Pyriproxifen, Tebufenozide, Teflubenzuron, Triflumuron,
Imidacloprid, Nitenpyram, N-[(6-Chloro-3-pyridinyl)methyl]-N'-cyano-N-methylethanimidamid (NI-25),
Abamectin, Amitrazin, Avermectin, Azadirachtin, Bensultap, Bacillus thuringiensis, Cyromazine, Diafenthiuron, Emamectin, Ethofenprox, Fenpyrad, Fipronil, Flufenprox, Lufenuron, Metaldehyd, Milbemectin, Pymetrozine, Tebufenpyrad, Triazuron,
Aldicarb, Bendiocarb, Benfuracarb, Carbofuran, Carbosulfan, Chlorethoxyfos, Cloethocarb, Disulfoton, Ethophrophos, Etrimphos, Fenamiphos, Fipronil, Fonofos, Fosthiazate, Furathiocarb, HCH, Isazophos, Isofenphos, Methiocarb, Monocrotophos, Nitenpyram, Oxamyl, Phorate, Phoxim, Prothiofos, Pyrachlofos, Sebufos, Silafluofen, Tebupirimphos, Tefluthrin, Terbufos, Thiodicarb, Thiafenox,
Azocyclotin, Butylpyridaben, Clofentezine, Cyhexatin, Diafenthiuron, Diethion, Emamectin, Fenazaquin, Fenbutatin Oxide, Fenothiocarb, Fenpropathrin, Fenpyrad, Fenpyroximate, Fluazinam, Fluazuron, Flucycloxuron, Flufenoxuron, Fluvalinate, Fubfenprox, Hexythiazox, Ivemectin, Methidathion, Monocrotophos, Moxidectin, Naled, Phosalone, Profenofos, Pyraclofos, Pyridaben, Pyrimidifen, Tebufenpyrad, Thuringiensin, Triarathene sowie 4-Bromo-2-(4-chlorophenyl)-1-(ethoxymethyl)-5-(trifluoromethyl)-1H-pyrrole-3-carbonitril (AC 303630).

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ektoparasiten und Endoparasiten) wie Schildzecken, Lederzecken, Räudemilben, Laufinilben, Fliegen (stechend und leckend), parasitierende Fliegenlarven, Läuse, Haarlinge, Federlinge, Flöhe und endoparasitisch lebende Würmer. Beispielsweise zeigen sie eine hervorragende Wirksamkeit gegen Zecken, wie beispielsweise Boophilus microplus.

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Die Herstellung und die Verwendung der erfindungsgemäßen Stoffe werden durch die folgenden Beispiele veranschaulicht.

### Herstellungsbeispiele

### Beispiel 1

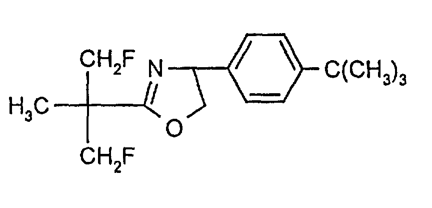

### (Verfahren c)

Zu 4,3 g (0,013 Mol) N-[2-Chlor-1-(4-t-butylphenyl)-ethyl]-2,2-bis(fluormethyl)-propionsäureamid in 100 ml Methanol werden unter Rückfluß 1,6 ml (0,026 Mol) 45 %-ige Natronlauge in 5 ml Wasser getropft. Man läßt das Reaktionsgemisch 1 Stunde unter Rückfluß nachrühren und engt anschließend durch Abdestillieren des Lösungsmittels ein. Der Rückstand wird mit Wasser verrührt, der Niederschlag abgesaugt und getrocknet.

Man erhält 3,8 g (99 % der Theorie) 2-(1,3-Difluor-2-methyl-prop-2-yl)-4-(4-t-butylphenyl)-1,3-oxazolin vom Schmelzpunkt 62-63°C.

### Herstellung der Ausgangsverbindung

### Beispiel (IV-1)

### (Stufe 1)

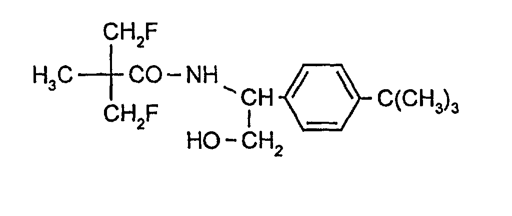

Zu 3,9 g (0,02 Mol) 2-Amino-2-(4-t-butylphenyl)-1-ethanol in 150 ml Ethylacetat gibt man 3,3 ml (0,024 Mol) Triethylamin und versetzt anschließend bei 0°C tropfenweise mit 3,7 g (0,024 Mol) 2,2-Bis(fluormethyl)-propionsäurechlorid. Danach läßt man auf Raumtemperatur erwärmen und rührt über Nacht nach. Anschließend wird der Niederschlag abgesaugt, das Filtrat mehrmals mit Wasser gewaschen, die organische Phase abgetrennt, über Natriumsulfat getrocknet, filtriert und eingeengt.

Man erhält 4,5 g (71,9 % der Theorie) N-[2-Hydroxy-1-(4-t-butylphenyl)-ethyl]-2,2-bis(fluormethyl)-propionsäureamid vom Schmelzpunkt 141°C.

### Beispiel (V-1)

### (Stufe 2)

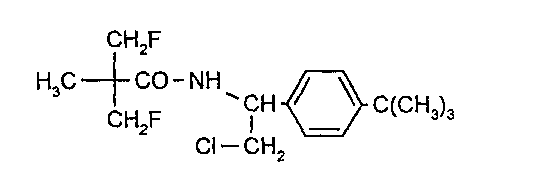

Zu 4,5 g (0,0144 Mol) N-[2-Hydroxy-1-(4-t-butylphenyl)-ethyl]-2,2-bis(fluormethyl)-propionsäureamid (vgl. Stufe 1) in 100ml Tetrachlorkohlenstoff werden 1,05ml (0,0144 Mol) Thionylchlorid gegeben. Man läßt das Reaktionsgemisch unter Rückfluß 1 Stunde nachreagieren. Nach Abdestillieren des Lösungsmittels erhält man 4,3 g (90 % der Theorie) N-[2-Chlor-1(4-t-butylphenyl)-ethyl]-2,2-bis(fluormethyl)-propionsäureamid vom Schmelzpunkt 98°C,

### Beispiel 2

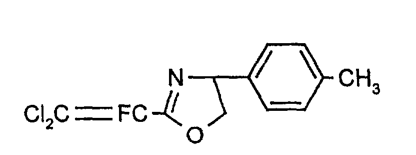

### (Verfahren c)

3,4 g (20 mmol) 2-(4-Methylphenyl)-2-aminoethanol werden in 50 ml abs. Tetrahydrofuran mit 2,8 ml Triethylamin versetzt und bei 0-10°C 3,6 g (20 mmol) 3,3-Dichlor-2-fluor-acrylsäurechlorid zugetropft. Nach einer Stunde Rühren bei Raumtemperatur wird der Niederschlag abgesaugt, das Filtrat eingeengt, der Rückstand in 50 ml abs. Toluol aufgenommen, mit 4,8 g Thionylchlorid versetzt und zwei Stunden unter Rückfluß erhitzt. Im Vakuum wird das Toluol und überschüssiges Thionylchlorid entfernt. Der Rückstand wird erneut in Toluol aufgenommen, portionsweise mit 3 g (22 mmol) Kalium-tert.-butylat versetzt und unter dünnschicht chromatographischer Kontrolle auf 50°C erwärmt. Die Reaktionsmischung wird mit Wasser gewaschen und die Toluolphase eingeengt. Der Rückstand wird an Kieselgel mit n-Hexan/Essigester 10:1 chromatographiert.

Man erhält 2,3 g (41 % der Theorie) 2-(2,2-Dichlor-1-fluorvinyl)-4-(4-methylphenyl) 1,3-oxazolin; ¹HNMR (500 MHz, CDCl₃) : 2,33 (s, 3H); 4,25 (t, 1H); 7,15 (m, 4H).

### Herstellung der Ausgangsverbindung

Es werden 515 g 2-Fluor-1,1,3,3,3-pentachlorpropen und 27 g Eisen-(III)-chlorid vorgelegt und bei 110°C innerhalb von 2 Stunden 52,6 g Wasser zugetropft. Man läßt 1 Stunde bei 110°C nachrühren, kühlt auf ca. 70°C ab, trennt durch Filtration die Feststoffe ab und tropft 330,2 ml Thionylchlorid zu. Nach ca. 2 Stunden Rückfluß werden das überschüssige Thionylchlorid und das Dichlorfluoracrylsäurechlorid destillativ getrennt.

Man erhält 233 g (90 % der Theorie) Dichlorfluoracrylsäurechlorid vom Siedepunkt 134°C.

In entsprechender Weise und gemäß den allgemeinen Angaben zur Herstellung erhält man die folgenden substituierten Oxazoline der Formel (Ia):

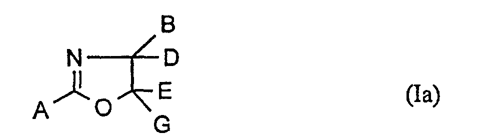

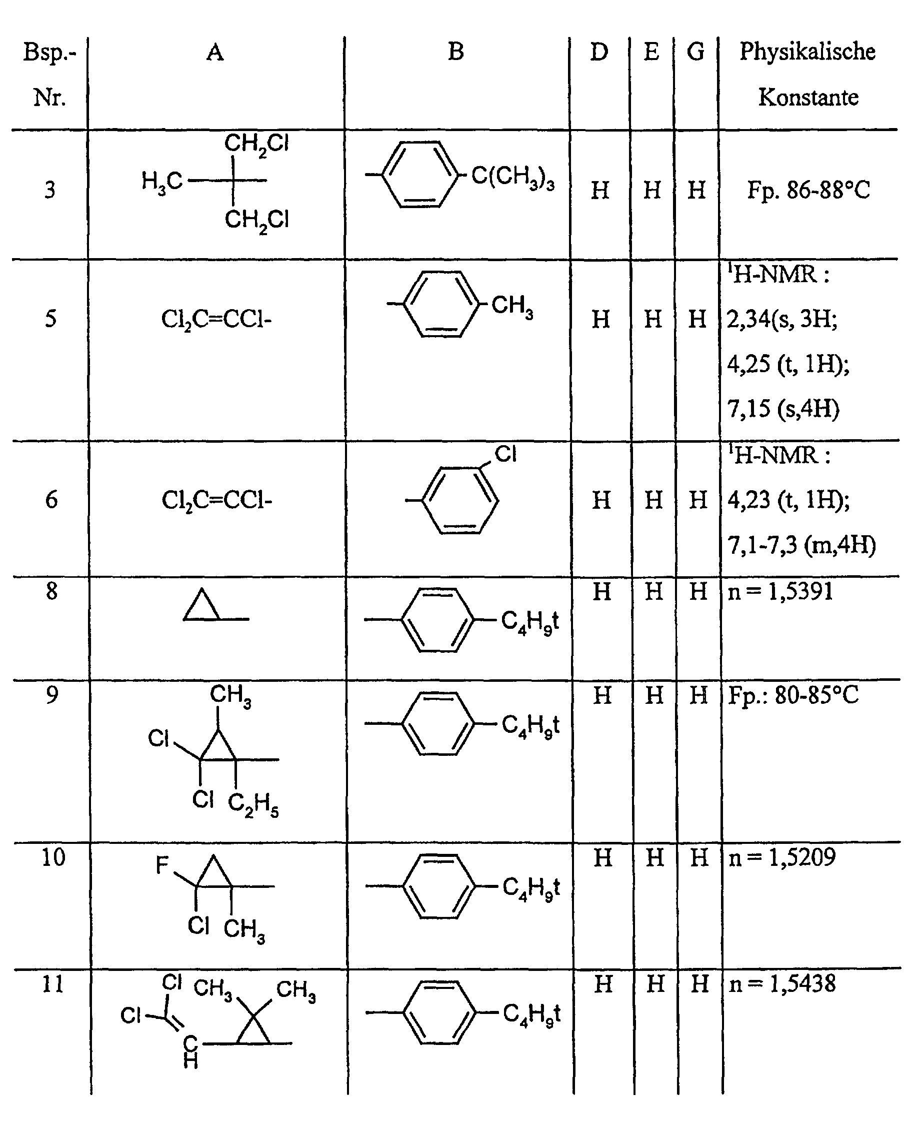

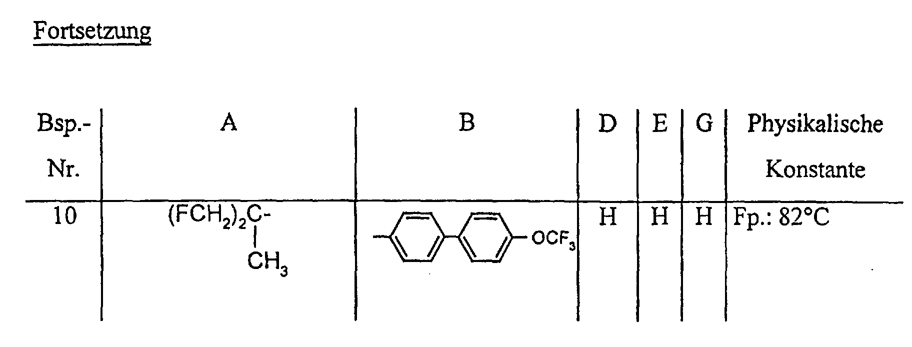

### Anwendungsbeispiele

### Beispiel A

**Tetranychus-Test** (OP-resistent/Tauchbehandlung)

| | |
|---|---|
| Lösungsmittel | 7 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit emulgatorhaltigem Wasser auf die gewünschten Konzentrationen.

Bohnenpflanzen (Phaseolus vulgaris), die stark von allen Entwicklungsstadien der gemeinen Spinnmilbe Tetranychus urticae befallen sind, werden in eine Wirkstoffzubereitung der gewünschten Konzentration getaucht.

Nach der gewünschten Zeit wird die Wirkung in % bestimmt. Dabei bedeutet 100%, daß alle Spinnmilben abgetötet wurden; 0% bedeutet, daß keine Spinnmilben abgetötet wurden.

In diesem Test bewirkten z.B. die Verbindungen gemäß den Herstellungsbeispielen 8 und 10 bei einer beispielhaften Wirkstoffkonzentration von 0,1 % eine Abtötung von mindestens 80 % nach 7 Tagen.

### Beispiel B

Spodoptera-Test

| | |
|---|---|
| Lösungsmittel | 7 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen des Eulenfalters (Spodoptera frugiperda) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

Bei diesem Test zeigt z.B. die Verbindung des Herstellungsbeispiels 1 bei einer beispielhaften Wirkstoffkonzentration von 0,1 % nach 7 Tagen einen Abtötungsgrad von 100%.

### Beispiel C

Plutella-Test

| | |
|---|---|
| Lösungsmittel | 7 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella maculipennis) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

Bei diesem Test zeigen z.B. die Verbindungen der Herstellungsbeispiele 1 und 10 bei einer beispielhaften Wirkstoffkonzentration von 0,1% nach 7 Tagen einen Abtötungsgrad von 100%.

### Beispiel D

Nephotettix-Test

| | |
|---|---|
| Lösungsmittel | 7 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Reiskeimlinge (Oryzae sativa) werden durch Tauchen in die Wirkstoffzubereitung der gewünschten Konzentration behandelt und mit Larven der Grünen Reiszikade (Nephotettix cincticeps) besetzt, solange die Keimlinge noch feucht sind.

Nach der gewünschten Zeit wird die Abtötung in % bestimmt. Dabei bedeutet 100 %, daß alle Zikaden abgetötet wurden; 0 % bedeutet, daß keine Zikaden abgetötet wurden.

Bei diesem Test zeigten z.B. die Verbindungen der Herstellungsbeispiele 8, 10 und 11 bei einer beispielhaften Wirkstoffkonzentration von 0,1% nach 7 Tagen einen Abtötungsgrad von mindestens 90%.

## Patentansprüche

1. Verwendung von substituierten Oxazolinen der Formel (I), 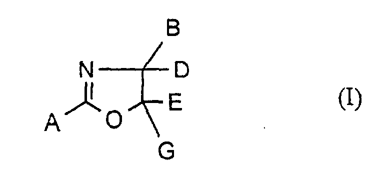 in welcher
A für C₁-C₈-Alkyl, das gegebenenfalls einfach oder mehrfach, gleich oder verschieden substituiert ist duch Halogen, Cyano, C₁-C₄-Alkoxy sowie durch jeweils gegebenenfalls einfach bis dreifach, gleich oder verschieden durch Halogen, C₁-C₄-Alkyl, C₁-C₄-Halogenalkyl, C₁-C₄-Alkoxy, C₁-C₄-Halogenalkoxy, C₁-C₄-Alkylthio und/oder C₁-C₄-Halogenalkylthio substituiertes Phenyl, Phenoxy, Benzyloxy, Phenylthio und Benzylthio,
für gegebenenfalls einfach oder mehrfach, gleich oder verschieden durch Halogen substituiertes C₂-C₈-Alkenyl,
für C₃-C₈-Cycloalkyl, das gegebenenfalls einfach oder mehrfach, gleich oder verschieden substituiert ist durch
Halogen, C₁-C₄-Alkyl, C₂-C₄-Alkenyl, C₁-C₄-Halogenalkyl oder C₂-C₄-Halogenalkenyl,
sowie für C₅-C₇-Cycloalkenyl steht, das gegebenenfalls einfach oder mehrfach, gleich oder verschieden substituiert ist durch
Halogen, C₁-C₄-Alkyl oder C₁-C₄-Halogenalkyl und
B für jeweils gegebenenfalls einfach bis vierfach, gleich oder verschieden substituiertes Phenyl, Benzyl Pheneth-1-yl, Pheneth-2-yl, Phenoxymethyl, Phenylthiomethyl, Phenoxyeth-1-yl, Phenylthioeth-1-yl, Phenoxyeth-2-yl oder Styryl steht, wobei jeweils als Phenylsubstituenten genannt seien
Halogen,
C₁-C₁₈-Alkyl,
C₁-C₈-Alkoxy-C₁-C₈-alkyl,
C₁-C₈-Halogenalkoxy,
C₁-C₄-Halogenalkyl,
C₁-C₁₈-Alkoxy, das gegebenenfalls durch weitere 1-3 Sauerstoffatome unterbrochen ist,
C₁-C₁₈-Alkylthio,
C₁-C₈-Halogenalkylthio,
3,4-Difluormethylendioxo,
3,4-Tetrafluorethylendioxo,
gegebenenfalls durch C₁-C₄-Alkyl, C₃-C₆-Cycloalkyl und/oder Halogen substituiertes Benzyliminooxymethyl,
jeweils gegebenenfalls durch C₁-C₆-Alkyl, C₁-C₆-Alkoxy, Cyclohexyl oder Phenyl substituiertes Cyclohexyl und Cyclohexyloxy;
gegebenenfalls einfach oder zweifach, gleich oder verschieden durch Halogen,
C₁-C₄-Alkyl oder C₁-C₄-Halogenalkyl substituiertes Pyridyloxy;
jeweils gegebenenfalls einfach bis dreifach, gleich oder verschieden durch
C₁-C₁₂-Alkyl, Halogen, C₁-C₄-Halogenalkyl, C₁-C₆-Alkoxy, C₁-C₆-Halogenalkoxy, C₁-C₆-Alkoxy-C₁-C₆-alkyl, C₁-C₆-Alkoxyethylenoxy, C₁-C₆-Alkylthio und/oder C₁-C₆-Halogenalkylthio substituiertes Phenyl, Benzyl, Phenoxy, Phenylthio, Benzyloxy und Benzylthio,
D für Wasserstoff oder Methyl steht;
E für Wasserstoff oder Methyl steht; und
G für Wasserstoff oder Methyl steht
zur Bekämpfung von tierischen Schädlingen, ausgenommen am tierischen Körper.

2. Verwendung von substituierten Oxazolinen der Formel (I) gemäß Anspruch 1,
in welcher
A für C₁-C₆-Alkyl, das gegebenenfalls einfach oder mehrfach, gleich oder verschieden substituiert ist durch
Fluor, Chlor, Brom, Cyano, C₁-C₂-Alkoxy sowie durch jeweils gegebenenfalls einfach oder zweifach, gleich oder verschieden durch Fluor, Chlor, C₁-C₄-Alkyl, C₁-C₂-Halogenalkyl, C₁-C₂-Alkoxy, C₁-C₂-Halogenalkoxy, C₁-C₂-Alkylthio und/oder C₁-C₂-Halogenalkylthio substituiertes Phenyl, Phenoxy, Benzyloxy, Phenylthio und Benzylthio;
für gegebenenfalls einfach oder mehrfach, gleich oder verschieden durch Fluor, Chlor und/oder Brom substituiertes C₂-C₆-Alkenyl;
für C₃-C₈-Cycloalkyl, das gegebenenfalls einfach oder mehrfach, gleich oder verschieden substituiert ist durch Fluor, Chlor, C₁-C₄-Alkyl, C₂-C₄-Alkenyl, sowie jeweils durch Fluor und/oder Chlor substituiertes C₁-C₂-Alkyl oder C₂-C₄-Alkenyl;
sowie für C₅-C₇-Cycloalkenyl steht, das gegebenenfalls einfach oder mehrfach, gleich oder verschieden substituiert ist durch
Fluor, Chlor, C₁-C₄-Alkyl sowie durch Fluor und/oder Chlor substituiertes C₁-C₂-Alkyl und
B für jeweils gegebenenfalls einfach bis vierfach, gleich oder verschieden substituiertes Phenyl, Benzyl, Pheneth-1-yl, Pheneth-2-yl, Phenoxymethyl, Phenylthiomethyl, Phenoxyeth-1-yl, Phenoxyeth-2-yl, Phenylthioeth-1-yl oder Styryl steht,
wobei jeweils als Phenylsubstituenten genannt seien
F, Cl, Br,
C₁-C₁₈-Alkyl,
C₁-C₆-Alkoxy-C₁-C₈-alkyl,
einfach bis sechsfach, gleich oder verschieden durch F und/oder Cl substituiertes C₁-C₈-Alkoxy,
einfach bis fünffach, gleich oder verschieden durch F und/oder Cl substituiertes C₁-C₂-Alkyl,
C₁-C₁₈-Alkoxy und -(OC₂H₄)₁₋₃-O-C₁-C₆-alkyl,
C₁-C₁₂-Alkylthio,
einfach bis sechsfach, gleich oder verschieden durch F und/oder Cl substituiertes C₁-C₈-Alkylthio,
3,4-Difluormethylendioxo,
3,4-Tetrafluorethylendioxo,
die Gruppierungen 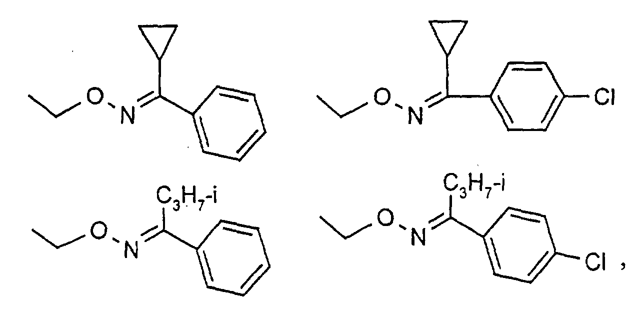 jeweils gegebenenfalls durch C₁-C₄-Alkyl, C₁-C₄-Alkoxy, Cyclohexyl oder Phenyl substituiertes Cyclohexyl und Cyclohexyloxy;
gegebenenfalls einfach oder zweifach, gleich oder verschieden durch F, Cl oder CF₃ substituiertes Pyridyloxy;
jeweils gegebenenfalls einfach bis dreifach, gleich oder verschieden durch C₁-C₁₂-Alkyl, F, Cl, Br, CF₃, C₁-C₄-Alkoxy, einfach bis sechsfach, gleich oder verschieden durch F und/oder Cl substituiertes C₁-C₄-Alkoxy, C₁-C₄-Alkoxy-C₁-C₄-alkyl, C₁-C₄-Alkoxy-ethylenoxy, C₁-C₄-Alkylthio, einfach bis sechsfach, gleich oder verschieden durch F und/oder Cl substituiertes C₁-C₄-Alkylthio substituiertes Phenyl, Benzyl, Phenoxy, Phenylthio, Benzyloxy und Benzylthio,
D für Wasserstoff oder Methyl steht;
E für Wasserstoff oder Methyl steht; und
G für Wasserstoff oder Methyl steht
zur Bekämpfung von tierischen Schädlingen ausgenommen am tierischen Körper.

3. Schädlingsbekämpfungsmittel, **gekennzeichnet durch** einen Gehalt an mindestens einer Verbindung der Formel (I) gemäß Anspruch 1, Streckmitteln und/oder oberflächenaktiven Mitteln.

4. Verfahren zur Bekämpfung von tierischen Schädlingen, **dadurch gekennzeichnet, daß** man Verbindungen der Formel (I) gemäß Anspruch 1 auf tierische Schädlinge und/oder deren Lebensraum ausgenommen den tierischen Körper einwirken läßt.

5. Verfahren zur Herstellung von Schädlingsbekämpfungsmitteln, **dadurch gekennzeichnet, daß** man Verbindungen der Formel (I) gemäß Anspruch 1 mit Streckmitteln und/oder oberflächenaktiven Mitteln vermischt.

6. Substituierte Oxazoline der Formel (Ia), 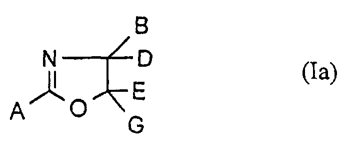 in welcher
A für C₁-C₈-Alkyl, das gegebenenfalls einfach oder mehrfach, gleich oder verschieden substituiert ist duch Halogen, Cyano, C₁-C₄-Alkoxy sowie durch jeweils gegebenenfalls einfach bis dreifach, gleich oder verschieden durch Halogen, C₁-C₄-Alkyl, C₁-C₄-Halogenalkyl, C₁-C₄-Alkoxy, C₁-C₄-Halogenalkoxy, C₁-C₄-Alkylthio und/oder C₁-C₄-Halogenalkylthio substituiertes Phenyl, Phenoxy, Benzyloxy, Phenylthio und Benzylthio,
für gegebenenfalls einfach oder mehrfach, gleich oder verschieden durch Halogen substituiertes C₂-C₈-Alkenyl,
für C₃-C₈-Cycloalkyl, das gegebenenfalls einfach oder mehrfach, gleich oder verschieden substituiert ist durch
Halogen, C₁-C₄-Alkyl, C₂-C₄-Alkenyl, C₁-C₄-Halogenalkyl oder C₂-C₄-Halogenalkenyl,
sowie für C₅-C₇-Cycloalkenyl steht, das gegebenenfalls einfach oder mehrfach, gleich oder verschieden substituiert ist durch Halogen, C₁-C₄-Alkyl oder C₁-C₄-Halogenalkyl und
B für jeweils einfach bis vierfach, gleich oder verschieden substituiertes Phenyl, Benzyl Pheneth-1-yl, Pheneth-2-yl, Phenoxymethyl, Phenylthiomethyl, Phenoxyeth-1-yl, Phenylthioeth-1-yl, Phenoxyeth-2-yl oder Styryl steht, wobei jeweils als Phenylsubstituenten genannt seien Halogen,
C₁-C₁₈-Alkyl,
C₁-C₈-Alkoxy-C₁-C₈-alkyl,
C₁-C₈-Halogenalkoxy,
C₁-C₄-Halogenalkyl,
C₁-C₁₈-Alkoxy, das gegebenenfalls durch weitere 1-3 Sauerstoffatome unterbrochen ist,
C₁-C₁₈-Alkylthio,
C₁-C₈-Halogenalkylthio,
3,4-Difluormethylendioxo,
3,4-Tetrafluorethylendioxo,
gegebenenfalls durch C₁-C₄-Alkyl, C₃-C₆-Cycloalkyl und/oder Halogen substituiertes Benzyliminooxymethyl,
jeweils gegebenenfalls durch C₁-C₆-Alkyl, C₁-C₆-Alkoxy, Cyclohexyl oder Phenyl substituiertes Cyclohexyl und Cyclohexyloxy;
gegebenenfalls einfach oder zweifach, gleich oder verschieden durch Halogen,
C₁-C₄-Alkyl oder C₁-C₄₋Halogenalkyl substituiertes Pyridyloxy;
jeweils einfach bis dreifach, gleich oder verschieden durch C₁-C₁₂-Alkyl, Halogen, C₁-C₄-Halogenalkyl, C₁-C₆-Alkoxy, C₁-C₆-Halogenalkoxy, C₁-C₆-Alkoxy-C₁-C₆-alkyl, C₁-C₆-Alkoxyethylenoxy, C₁-C₆-Alkylthio und/oder C₁-C₆-Halogenalkylthio substituiertes Phenoxy, Phenylthio, Benzyloxy und Benzylthio,
D für Wasserstoff oder Methyl steht;
E für Wasserstoff oder Methyl steht; und
G für Wasserstoff oder Methyl steht.

7. Substituierte Oxazoline der Formel (Ia) gemäß Anspruch 6,
in welcher
A für C₁-C₆-Alkyl, das gegebenenfalls einfach oder mehrfach, gleich oder verschieden substituiert ist durch
Fluor, Chlor, Brom, Cyano, C₁-C₂-Alkoxy sowie durch jeweils gegebenenfalls einfach oder zweifach, gleich oder verschieden durch Fluor, Chlor, C₁-C₄-Alkyl, C₁-C₂-Halogenalkyl, C₁-C₂-Alkoxy, C₁-C₂-Halogenalkoxy, C₁-C₂-Alkylthio und/oder C₁-C₂-Halogenalkylthio substituiertes Phenyl, Phenoxy, Benzyloxy, Phenylthio und Benzylthio;
für gegebenenfalls einfach oder mehrfach, gleich oder verschieden durch Fluor, Chlor und/oder Brom substituiertes C₂-C₆-Alkenyl;
für C₃-C₈-Cycloalkyl, das gegebenenfalls einfach oder mehrfach, gleich oder verschieden substituiert ist durch
Fluor, Chlor, C₁-C₄-Alkyl, C₂-C₄-Alkenyl, sowie jeweils durch Fluor und/oder Chlor substituiertes C₁-C₂-Alkyl oder C₂-C₄-Alkenyl;
sowie für C₅-C₇-Cycloalkenyl steht, das gegebenenfalls einfach oder mehrfach, gleich oder verschieden substituiert ist durch
Fluor, Chlor, C₁-C₄-Alkyl sowie durch Fluor und/oder Chlor substituiertes C₁-C₂-Alkyl und
B für jeweils einfach bis vierfach, gleich oder verschieden substituiertes Phenyl, Benzyl, Pheneth-1-yl, Pheneth-2-yl, Phenoxymethyl, Phenylthiomethyl, Phenoxyeth-1-yl, Phenoxyeth-2-yl, Phenylthioeth-1-yl oder Styryl steht,
wobei jeweils als Phenylsubstituenten genannt seien
F, Cl, Br,
C₁-C₁₈-Alkyl,
C₁-C₆-Alkoxy-C₁-C₈-alkyl,
einfach bis sechsfach, gleich oder verschieden durch F und/oder Cl substituiertes C₁-C₈-Alkoxy,
einfach bis fünffach, gleich oder verschieden durch F und/oder Cl substituiertes C₁-C₂-Alkyl,
C₁-C₁₈-Alkoxy und -(OC₂H₄)₁₋₃-O-C₁-C₆-alkyl,
C₁-C₁₂-Alkylthio,
einfach bis sechsfach, gleich oder verschieden durch F und/oder Cl substituiertes C₁-C₈-Alkylthio,
3,4-Difluormethylendioxo,
3,4-Tetrafluorethylendioxo,
die Gruppierungen 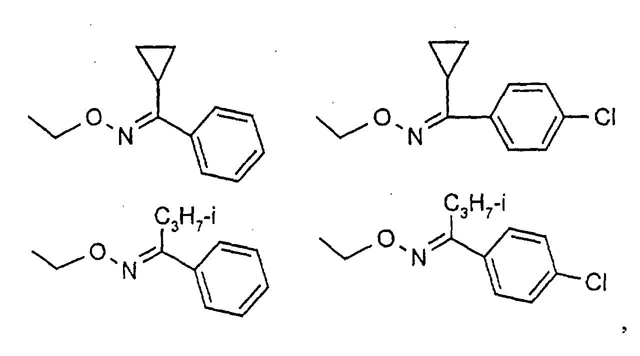 jeweils gegebenenfalls durch C₁-C₄-Alkyl, C₁-C₄-Alkoxy, Cyclohexyl oder Phenyl substituiertes Cyclohexyl und Cyclohexyloxy;
gegebenenfalls einfach oder zweifach, gleich oder verschieden durch F, Cl oder CF₃ substituiertes Pyridyloxy;
jeweils einfach bis dreifach, gleich oder verschieden durch C₁-C₁₂-Alkyl, F, Cl, Br, CF₃, C₁-C₄-Alkoxy, einfach bis sechsfach, gleich oder verschieden durch F und/oder Cl substituiertes C₁-C₄-Alkoxy, C₁-C₄-Alkoxy-C₁-C₄-alkyl, C₁-C₄-Alkoxy-ethylenoxy, C₁-C₄-Alkylthio, einfach bis sechsfach, gleich oder verschieden durch F und/oder Cl substituiertes C₁-C₄-Alkylthio substituiertes Phenoxy, Phenylthio, Benzyloxy und Benzylthio,
D für Wasserstoff oder Methyl steht;
E für Wasserstoff oder Methyl steht; und
G für Wasserstoff oder Methyl steht.

8. Verfahren zur Herstellung von substituierten Oxazolinen der Formel (Ia) gemäß Anspruch 6, **dadurch gekennzeichnet, daß** man
a) Aminoalkohole der Formel (II) 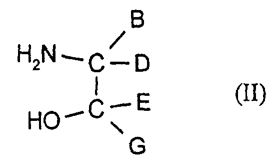 in welcher
B, D, E und G die in Anspruch 6 angegebenen Bedeutungen haben,
mit einer Carbonsäure der Formel (III)
A-COOH (III)
in welcher
A die in Anspruch 6 angegebene Bedeutung hat,
mit einem wasser-entziehenden Mittel und gegebenenfalls in Gegenwart eines Verdünnungsmittels umsetzt;
oder
b) Amidalkohole der Formel (IV) 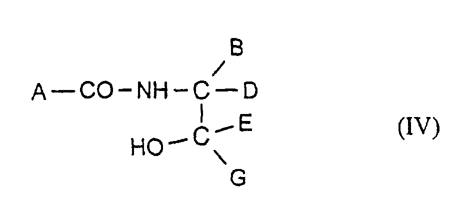 in welcher
A, B, D, E und G die oben angegebenen Bedeutungen haben,
mit einem wasser-entziehenden Mittel, gegebenenfalls in Gegenwart eines Verdünnungsmittels umsetzt;
oder
c) Amid-Derivate der Formel (V) 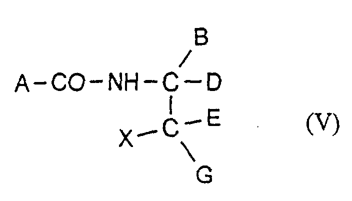 in welcher
A, B, D, E und G die oben angegebenen Bedeutungen haben; und
X für eine Abgangsgruppe steht,
mit einer Base, gegebenenfalls in Gegenwart eines Verdünnungsmittels umsetzt.

## Claims

1. Use of substituted oxazolines of the formula (I) 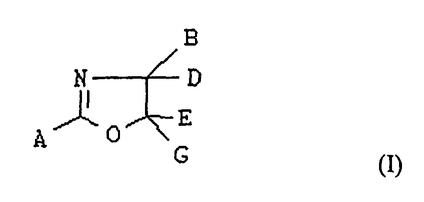 in which
A represents C₁-C₈-alkyl which is optionally substituted one or more times by identical or different substituents chosen from halogen, cyano and C₁-C₄-alkoxy and from phenyl, phenoxy, benzyloxy, phenylthio and benzylthio, each of which is optionally substituted one to three times by identical or different substituents chosen from halogen, C₁-C₄-alkyl, C₁-C₄-halogenoalkyl, C₁-C₄-alkoxy, C₁-C₄-halogenoalkoxy, C₁-C₄-alkylthio and/or C₁-C₄-halogenoalkylthio,
represents C₂-C₈-alkenyl which is optionally substituted one or more times by identical or different halogen substituents,
represents C₃-C₈-cycloalkyl which is optionally substituted one or more times by identical or different substituents chosen from halogen, C₁-C₄-alkyl, C₂-C₄-alkenyl, C₁-C₄-halogenoalkyl or C₂-C₄-halogenoalkenyl,
and represents C₅-C₇-cycloalkenyl which is optionally substituted one or more times by identical or different substituents chosen from halogen, C₁-C₄-alkyl and C₁-C₄-halogenoalkyl and
B represents phenyl, benzyl, pheneth-1-yl, pheneth-2-yl, phenoxymethyl, phenylthiomethyl, phenoxyeth-1-yl, phenylthioeth-1-yl, phenoxyeth-2-yl or styryl, each of which is optionally substituted one to four times by identical or different substituents, phenyl substituents which may be mentioned being in each case
halogen,
C₁-C₁₈-alkyl,
C₁-C₈-alkoxy-C₁-C₈-alkyl,
C₁-C₈-halogenoalkoxy,
C₁-C₄-halogenoalkyl,
C₁-C₁₈-alkoxy which is optionally interrupted by 1-3 further oxygen atoms,
C₁-C₁₈-alkylthio,
C₁-C₈-halogenoalkylthio,
3,4-difluoromethylenedioxo,
3,4-tetrafluoroethylenedioxo,
benzyliminooxymethyl which is optionally substituted by C₁-C₄-alkyl, C₃-C₆-cycloalkyl and/or halogen,
cyclohexyl and cyclohexyloxy, each of which is optionally substituted by C₁-C₆-alkyl, C₁-C₆-alkoxy, cyclohexyl or phenyl;
pyridyloxy which is optionally substituted once or twice by identical or different substituents chosen from halogen, C₁-C₄-alkyl or C₁-C₄-halogenoalkyl;
phenyl, benzyl, phenoxy, phenylthio, benzyloxy and benzylthio, each of which are optionally substituted one to three times by identical or different substituents chosen from C₁-C₁₂-alkyl, halogen, C₁-C₄-halogenoalkyl, C₁-C₆-alkoxy, C₁-C₆-halogenoalkoxy, C₁-C₆-alkoxy-C₁-C₆-alkyl, C₁-C₆-alkoxy-ethyleneoxy, C₁-C₆alkylthio and/or C₁-C₆-halogenoalkylthio,
D represents hydrogen or methyl;
E represents hydrogen or methyl; and
G represents hydrogen or methyl,
for combating animal pests, except on animal bodies.

2. Use of substituted oxazolines of the formula (I) according to Claim 1,
in which
A represents C₁-C₆-alkyl
which is optionally substituted one or more times by identical or different substituents chosen from fluorine, chlorine, bromine, cyano and C₁-C₂-alkoxy and by phenyl, phenoxy, benzyloxy, phenylthio and benzylthio, each of which is optionally substituted once or twice by identical or different substituents chosen from fluorine, chlorine, C₁-C₄-alkyl, C₁-C₂-halogenoalkyl, C₁-C₂-alkoxy, C₁-C₂-halogenoalkoxy, C₁-C₂-alkylthio and/or C₁-C₂-halogenoalkylthio;
represents C₂-C₆-alkenyl which is optionally substituted one or more times by identical or different substituents chosen from fluorine, chlorine and/or bromine;
represents C₃-C₈-cycloalkyl which is optionally substituted one or more times by identical or different substituents chosen from fluorine, chlorine, C₁-C₄-alkyl and C₂-C₄-alkenyl, and by C₁-C₂-alkyl or C₂-C₄-alkenyl, each of which is substituted by fluorine and/or chlorine;
and represents C₅-C₇-cycloalkenyl which is optionally substituted one or more times by identical or different substituents chosen from fluorine, chlorine and C₁-C₄-alkyl and from C₁-C₂-alkyl which is substituted by fluorine and/or chlorine and
B represents phenyl, benzyl, pheneth-1-yl, pheneth-2-yl, phenoxymethyl, phenylthiomethyl, phenoxyeth-1-yl, phenoxyeth-2-yl, phenylthioeth-1-yl or styryl, each of which is optionally substituted one to four times by identical or different substituents,
substituents for phenyl which may be mentioned being in each case F, Cl, Br,
C₁-C₁₈-alkyl,
C₁-C₆-alkoxy-C₁-C₈-alkyl,
C₁-C₈-alkoxy which is substituted one to six times by identical or different substituents chosen from F and/or Cl,
C₁-C₂-alkyl which is substituted one to five times by identical or different substituents chosen from F and/or Cl,
C₁-C₁₈-alkoxy and -(OC₂H₄)₁₋₃-O-C₁-C₆-alkyl,
C₁-C₁₂-alkylthio,
C₁-C₈-alkylthio which is substituted one to six times by identical or different substituents chosen from F and/or Cl,
3,4-difluoromethylenedioxo,
3,4-tetrafluoroethylenedioxo,
the groups 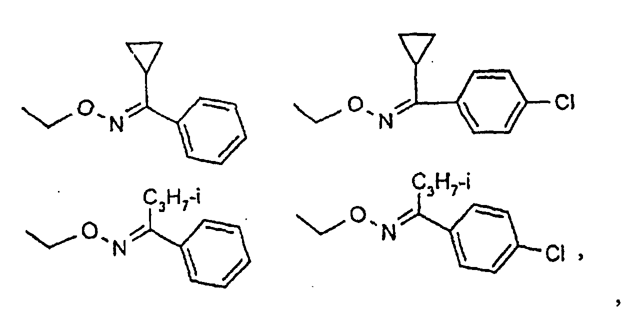 cyclohexyl and cyclohexyloxy, each of which is optionally substituted by C₁-C₄-alkyl, C₁-C₄-alkoxy, cyclohexyl or phenyl;
pyridyloxy which is optionally substituted once or twice by identical or different substituents chosen from F, Cl or CF₃;
phenoxy, phenylthio, benzyloxy and benzylthio, each of which is substituted one to three times by identical or different substituents chosen from C₁-C₁₂-alkyl, F, Cl, Br, CF₃ and C₁-C₄-alkoxy, C₁-C₄-alkoxy which is substituted one to six times by identical or different substituents chosen from F and/or Cl, C₁-C₄-alkoxy-C₁-C₄-alkyl, C₁-C₄-alkoxyethyleneoxy, C₁-C₄-alkylthio, or C₁-C₄-alkylthio which is substituted one to six times by identical or different substituents chosen from F and/or Cl,
D represents hydrogen or methyl;
E represents hydrogen or methyl; and
G represents hydrogen or methyl,
for combating animal pests, except on animal bodies.

3. Composition for combating pests, **characterized in that** it contains at least one compound of the formula (I) according to Claim 1, extenders and/or surface-active agents.

4. Method of combating animal pests, **characterized in that** compounds of the formula (I) according to Claim 1 are caused to act on animal pests and/or their habitat, except animal bodies.

5. Process for the preparation of compositions for combating pests, **characterized in that** compounds of the formula (I) according to Claim 1 are mixed with extenders and/or surface-active agents.

6. Substituted oxazolines of the formula (Ia), 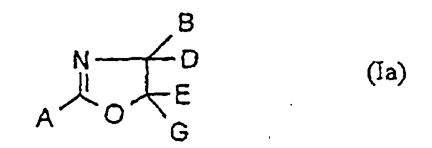 in which
A represents C₁-C₈-alkyl which is optionally substituted one or more times by identical or different substituents chosen from halogen, cyano and C₁-C₄-alkoxy and from phenyl, phenoxy, benzyloxy, phenylthio and benzylthio, each of which is optionally substituted one to three times by identical or different substituents chosen from halogen, C₁-C₄-alkyl, C₁-C₄-halogeno-alkyl, C₁-C₄-alkoxy, C₁-C₄-halogenoalkoxy, C₁-C₄-alkylthio and/or C₁-C₄-halogenoalkylthio,
represents C₂-C₈-alkenyl which is optionally substituted one or more times by identical or different halogen substituents,
represents C₃-C₈-cycloalkyl which is optionally substituted one or more times by identical or different substituents chosen from halogen, C₁-C₄-alkyl, C₂-C₄-alkenyl, C₁-C₄-halogenoalkyl or C₂-C₄-halogenoalkenyl,
and represents C₅-C₇-cycloalkenyl which is optionally substituted one or more times by identical or different substituents chosen from halogen, C₁-C₄-alkyl and C₁-C₄-halogenoalkyl and
B represents phenyl, benzyl, pheneth-1-yl, pheneth-2-yl, phenoxymethyl, phenylthiomethyl, phenoxyeth-1-yl, phenylthioeth-1-yl, phenoxyeth-2-yl or styryl, each of which is substituted one to four times by identical or different substituents, phenyl substituents which may be mentioned being in each case
halogen,
C₁-C₁₈-alkyl,
C₁-C₈-alkoxy-C₁-C₈-alkyl,
C₁-C₈-halogenoalkoxy,
C₁-C₄-halogenoalkyl,
C₁-C₁₈-alkoxy which is optionally interrupted by
1-3 further oxygen atoms,
C₁-C₁₈-alkylthio,
C₁-C₈-halogenoalkylthio,
3,4-difluoromethylenedioxo,
3,4-tetrafluoroethylenedioxo,
benzyliminooxymethyl which is optionally substituted by C₁-C₄-alkyl, C₃-C₆-cycloalkyl and/or
halogen,
cyclohexyl and cyclohexyloxy, each of which is optionally substituted by C₁-C₆-alkyl, C₁-C₆-alkoxy, cyclohexyl or phenyl;
pyridyloxy which is optionally substituted once or twice by identical or different substituents chosen from halogen, C₁-C₄-alkyl or C₁-C₄-halogenoalkyl;
phenoxy, phenylthio, benzyloxy and benzylthio, each of which are substituted one to three times by identical or different substituents chosen from C₁-C₁₂-alkyl, halogen, C₁-C₄-halogenoalkyl, C₁-C₆-alkoxy C₁-C₆-halogenoalkoxy, C₁-C₆-alkoxy-C₁-C₆-alkyl, C₁-C₆-alkoxy-ethyleneoxy, C₁-C₆alkylthio and/or C₁-C₆-halogenoalkylthio,
D represents hydrogen or methyl;
E represents hydrogen or methyl; and
G represents hydrogen or methyl.

7. Substituted oxazolines of the formula (Ia) according to Claim 6,
in which
A represents C₁-C₆-alkyl which is optionally substituted one or more times by identical or different substituents chosen from fluorine, chlorine, bromine, cyano and C₁-C₂-alkoxy and by phenyl, phenoxy, benzyloxy, phenylthio and benzylthio, each of which is optionally substituted once or twice by identical or different substituents chosen from fluorine, chlorine, C₁-C₄-alkyl, C₁-C₂-halogenoalkyl, C₁-C₂-alkoxy, C₁-C₂-halogenoalkoxy, C₁-C₂-alkylthio and/or C₁-C₂-halogenoalkylthio;
represents C₂-C₆-alkenyl which is optionally substituted one or more times by identical or different substituents chosen from fluorine, chlorine and/or bromine;
represents C₃-C₈-cycloalkyl which is optionally substituted one or more times by identical or different substituents chosen from fluorine, chlorine, C₁-C₄-alkyl and C₂-C₄-alkenyl, and by C₁-C₂-alkyl or C₂-C₄-alkenyl, each of which is substituted by fluorine and/or chlorine;
and represents C₅-C₇-cycloalkenyl which is optionally substituted one or more times by identical or different substituents chosen from fluorine, chlorine and C₁-C₄-alkyl and from C₁-C₂-alkyl which is substituted by fluorine and/or chlorine and
B represents phenyl, benzyl, pheneth-1-yl, pheneth-2-yl, phenoxymethyl, phenylthiomethyl, phenoxyeth-1-yl, phenoxyeth-2-yl, phenylthioeth-1-yl or styryl, each of which is substituted one to four times by identical or different substituents,
substituents for phenyl which may be mentioned
being in each case
F, Cl, Br,
C₁-C₁₈-alkyl,
C₁-C₆-alkoxy-C₁-C₈-alkyl,
C₁-C₈-alkoxy which is substituted one to six times by identical or different substituents chosen from F and/or Cl,
C₁-C₂-alkyl which is substituted one to five times by identical or different substituents chosen from F and/or Cl,
C₁-C₁₈-alkoxy and -(OC₂H₄)₁₋₃-O-C₁-C₆-alkyl,
C₁-C₁₂-alkylthio,
C₁-C₈-alkylthio which is substituted one to six times by identical or different substituents chosen from F and/or Cl,
3,4-difluoromethylenedioxo,
3,4-tetrafluoroethylenedioxo,
the groups 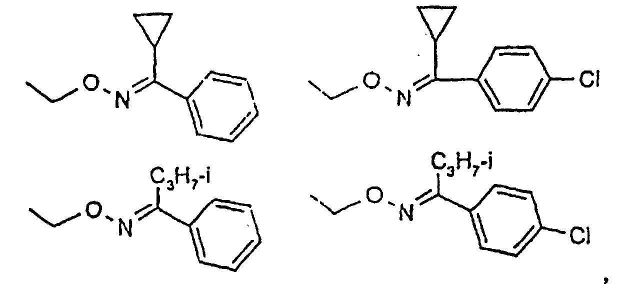 cyclohexyl and cyclohexyloxy, each of which is optionally substituted by C₁-C₄-alkyl, C₁-C₄-alkoxy, cyclohexyl or phenyl;
pyridyloxy which is optionally substituted once or twice by identical or different substituents chosen from F, C₁ or CF₃;
phenoxy, phenylthio, benzyloxy and benzylthio, each of which is substituted one to three times by identical or different substituents chosen from C₁-C₁₂-alkyl, F, Cl, Br, CF₃ and C₁-C₄-alkoxy, C₁-C₄-alkoxy which is substituted one to six times by identical or different substituents chosen from F and/or Cl, C₁-C₄-alkoxy-C₁-C₄-alkyl, C₁-C₄-alkoxyethyleneoxy, C₁-C₄-alkylthio, or C₁-C₄-alkylthio which is substituted one to six times by identical or different substituents chosen from F and/or Cl,
D represents hydrogen or methyl;
E represents hydrogen or methyl; and
G represents hydrogen or methyl.

8. Process for the preparation of substituted oxazolines of the formula (Ia) according to Claim 6, **characterized in that**
a) amino alcohols of the formula (II) 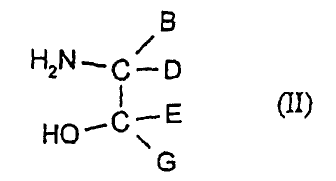 in which
B, D, E and G have the meanings given in Claim 6
are reacted with a carboxylic acid of the formula (III)
A-COOH (III)
in which
A has the meaning given in Claim 6,
with a dehydrating agent and, if desired, in the presence of a diluent;
or
b) amido alcohols of the formula (IV) 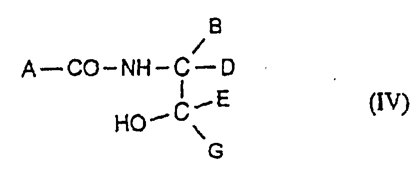 in which
A, B, D, E and G have the meanings given above
are reacted with a dehydrating agent, if desired in the presence of a diluent;
or
c) amide derivatives of the formula (V) 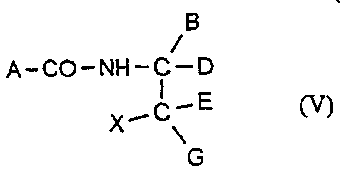 in which
A, B, D, E, and G have the meanings given above and
X represents a leaving group
are reacted with a base, if desired in the presence of a diluent.

## Revendications

1. Utilisation d'oxazolines substituées de formule (I) : 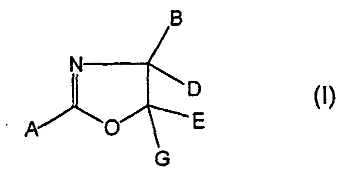 dans laquelle :
A représente un groupe alcoyle en C₁-C₈, qui est facultativement substitué une ou plusieurs fois, de manière identique ou différente, par un halogène, un groupe cyano, alcoxy en C₁-C₄ ainsi que par un groupe phényle, phénoxy, benzyloxy, phénylthio et benzylthio, chaque fois facultativement substitués une à trois fois, de manière identique ou différente, par un halogène, un groupe alcoyle en C₁-C₄, halogénoalcoyle en C₁-C₄, alcoxy en C₁-C₄, halogénoalcoxy en C₁-C₄, alcoylthio en C₁-C₄ et/ou halogénoalcoylthio en C₁-C₄,
A représente également un groupe alcényle en C₂-C₈, qui est facultativement substitué une ou plusieurs fois, de manière identique ou différente, par un halogène,
A représente aussi un groupe cycloalcoyle en C₃-C₈, qui est facultativement substitué une ou plusieurs fois, de manière identique ou différente, par un halogène, un groupe alcoyle en C₁-C₄, alcényle en C₂-C₄, halogénoalcoyle en C₁-C₄, halogénoalcényle en C₂-C₄,
et A représente aussi un groupe cycloalcényle en C₅-C₇, qui est facultativement substitué une ou plusieurs fois, de manière identique ou différente, par un halogène, un groupe alcoyle en C₁-C₄, ou halogénoalcoyle en C₁-C₄;
B représente un groupe phényle, benzyle, phénéth-1-yle, phénéth-2-yle, phénoxyméthyle, phénylthiométhyle, phénoxyéth-1-yle, phénylthioéth-1-yle, phénoxyéth-2-yle ou styryle, chaque fois facultativement une à quatre fois substitués, de manière identique ou différente, où l'on citera comme substituants sur les radicaux phényle :
halogène,
alcoyle en C₁-C₁₈,
(alcoxy en C₁-C₈)alcoyle en C₁-C₈,
halogénoalcoxy en C₁-C₈,
halogénoalcoyle en C₁-C₄,
alcoxy en C₁-C₁₈, qui est facultativement interrompu par 1 à 3 atomes d'oxygène,
alcoylthio en C₁-C₁₈,
halogénoalcoylthio en C₁-C₈,
3,4-difluorométhylènedioxo,
3,4-tétrafluoroéthylènedioxo,
benzyliminooxyméthyle, facultativement substitué par un groupe alcoyle en C₁-C₄, cycloalcoyle en C₃-C₆ et/ou un halogène,
cyclohexyle et cyclohexyloxy chaque fois facultativement substitués par un groupe alcoyle en C₁-C₆, alcoxy en C₁-C₆, cyclohexyle ou phényle ;
pyridyloxy facultativement substitué une ou deux fois, de manière identique ou différente, par un halogène, un groupe alcoyle en C₁-C₄ ou halogénoalcoyle en C₁-C₄ ;
phényle, benzyle, phénoxy, phénylthio, benzyloxy et benzylthio, chaque fois facultativement substitués une à trois fois, de manière identique ou différente, par un groupe alcoyle en C₁-C₁₂, un halogène, un groupe halogénoalcoyle en C₁-C₄, alcoxy en C₁-C₆, halogénoalcoxy en C₁-C₆, (alcoxy en C₁-C₆)alcoyle en C₁-C₆, (alcoxy en C₁-C₆)éthylèneoxy, alcoylthio en C₁-C₆ et/ou halogénoalcoylthio en C₁-C₆ ;
D représente l'atome d'hydrogène ou un radical méthyle ;
E représente l'atome d'hydrogène ou un radical méthyle, et
G représente l'atome d'hydrogène ou un radical méthyle ;
pour lutter contre les parasites animaux, excepté ceux présents sur le corps des animaux.

2. Utilisation d'oxazolines substituées suivant la revendication 1, où
A représente un groupe alcoyle en C₁-C₆, qui est facultativement substitué une ou plusieurs fois, de manière identique ou différente, par le fluor, le chlore, le brome, un groupe cyano, alcoxy en C₁-C₂ ainsi que par un groupe phényle, phénoxy, benzyloxy, phénylthio et benzylthio, chaque fois facultativement substitués une ou deux fois, de manière identique ou différente, par le fluor, le chlore, un groupe alcoyle en C₁-C₄, halogénoalcoyle en C₁-C₂, alcoxy en C₁-C₂, halogénoalcoxy en C₁-C₂, alcoylthio en C₁-C₂ et/ou halogénoalcoylthio en C₁-C₂,
A représente également un groupe alcényle en C₂-C₆, qui est facultativement substitué une ou plusieurs fois, de manière identique ou différente, par le fluor, le chlore et/ou le brome,
A représente aussi un groupe cycloalcoyle en C₃-C₈, qui est facultativement substitué une ou plusieurs fois, de manière identique ou différente, par le fluor, le chlore, un groupe alcoyle en C₁-C₄, alcényle en C₂-C₄, ainsi que alcoyle en C₁-C₂ ou alcényle en C₂-C₄ chaque fois substitué par le fluor et/ou le chlore ;
et A représente aussi un groupe cycloalcényle en C₅-C₇, qui est facultativement substitué une ou plusieurs fois, de manière identique ou différente, par le fluor, le chlore, un groupe alcoyle en C₁-C₄, ainsi que alcoyle en C₁-C₂ substitué par le fluor et/ou le chlore ;
B représente un groupe phényle, benzyle, phénéth-1-yle, phénéth-2-yle, phénoxyméthyle, phénylthiométhyle, phénoxyéth-1-yle, phénoxyéth-2-yle, phénylthioéth-1-yle ou styryle, chaque fois facultativement une à quatre fois substitués, de manière identique ou différente, où l'on citera comme substituants sur les radicaux phényle :
F, Cl, Br,
alcoyle en C₁-C₁₈,
(alcoxy en C₁-C₆)alcoyle en C₁-C₈,
alcoxy en C₁-C₈ substitué une à six fois, de manière identique ou différente par F et/ou Cl,
alcoyle en C₁-C₂ substitué une à cinq fois, de manière identique ou différente par F et/ou Cl,
alcoxy en C₁-C₁₈ et-(OC₂H₄)₁₋₃-O-alcoyle en C₁-C₆,
alcoylthio en C₁-C₁₂,
alcoylthio en C₁-C₈ substitué une à six fois, de manière identique ou différente par F et/ou Cl,
3,4-difluorométhylènedioxo,
3,4-tétrafluoroéthylènedioxo,
les groupements : 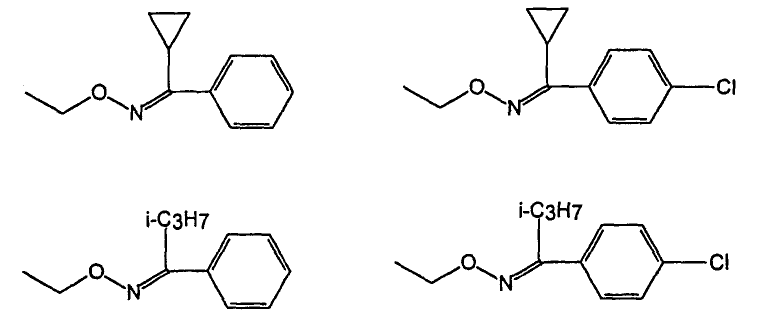
cyclohexyle et cyclohexyloxy chaque fois facultativement substitués par un groupe alcoyle en C₁-C₄, alcoxy en C₁-C₄, cyclohexyle ou phényle ;
pyridyloxy facultativement substitué une ou deux fois, de manière identique ou différente, par F, Cl ou CF₃;
phényle, benzyle, phénoxy, phénylthio, benzyloxy et benzylthio, chaque fois facultativement substitués une à trois fois, de manière identique ou différente, par un groupe alcoyle en C₁-C₁₂, F, Cl, Br, CF₃, alcoxy en C₁-C₄, alcoxy en C₁-C₄ substitué une à six fois, de manière identique ou différente par F et/ou Cl, (alcoxy en C₁-C₄)alcoyle en C₁-C₄, (alcoxy en C₁-C₄)éthylèneoxy, alcoylthio en C₁-C₄ et/ou alcoylthio en C₁-C₄ substitué une à six fois, de manière identique ou différente par F et/ou Cl ;
D représente l'atome d'hydrogène ou un radical méthyle ;
E représente l'atome d'hydrogène ou un radical méthyle, et
G représente l'atome d'hydrogène ou un radical méthylé ;
pour lutter contre les parasites animaux, excepté ceux présents sur le corps des animaux.

3. Agent parasiticide, **caractérisé par** la présence d'au moins un composé de formule (I) suivant la revendication 1, un diluant et/ou des agents tensioactifs.

4. Procédé de lutte contre les parasites animaux, **caractérisé en ce que** l'on fait agir les composés de formule (I) suivant la revendication 1, sur les parasites animaux et/ou leur habitat, excepté les corps animaux.

5. Procédé de préparation d'agents parasiticides, **caractérisé en ce que** l'on mélange des composés de formule (I) suivant la revendication 1, avec des diluants et/ou agents tensioactifs.

6. Oxazolines substituées de formule (Ia) : 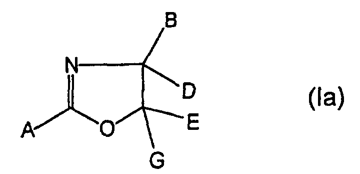 dans laquelle :
A représente un groupe alcoyle en C₁-C₈, qui est facultativement substitué une ou plusieurs fois, de manière identique ou différente, par un halogène, un groupe cyano, alcoxy en C₁-C₄ ainsi que par un groupe phényle, phénoxy, benzyloxy, phénylthio et benzylthio, chaque fois facultativement substitués une à trois fois, de manière identique ou différente, par un halogène, un groupe alcoyle en C₁-C₄, halogénoalcoyle en C₁-C₄, alcoxy en C₁-C₄, halogénoalcoxy en C₁-C₄, alcoylthio en C₁-C₄ et/ou halogénoalcoylthio en C₁-C₄,
A représente également un groupe alcényle en C₂-C₈, qui est facultativement substitué une ou plusieurs fois, de manière identique ou différente, par un halogène,
A représente aussi un groupe cycloalcoyle en C₃-C₈, qui est facultativement substitué une ou plusieurs fois, de manière identique ou différente, par un halogène, un groupe alcoyle en C₁-C₄, alcényle en C₂-C₄, halogénoalcoyle en C₁-C₄, halogénoalcényle en C₂-C₄,
et A représente aussi un groupe cycloalcényle en C₅-C₇, qui est facultativement substitué une ou plusieurs fois, de manière identique ou différente, par un halogène, un groupe alcoyle en C₁-C₄, ou halogénoalcoyle en C₁-C₄;
B représente un groupe phényle, benzyle, phénéth-1-yle, phénéth-2-yle, phénoxyméthyle, phénylthiométhyle, phénoxyéth-1-yle, phénylthioéth-1-yle, phénoxyéth-2-yle ou styryle, chaque fois facultativement une à quatre fois substitués, de manière identique ou différente, où l'on citera comme substituants sur les radicaux phényle :
halogène,
alcoyle en C₁-C₁₈,
(alcoxy en C₁-C₈)alcoyle en C₁-C₈,
halogénoalcoxy en C₁-C₈,
halogénoalcoyle en C₁-C₄,
alcoxy en C₁-C₁₈, qui est facultativement interrompu par 1 à 3 atomes d'oxygène,
alcoylthio en C₁-C₁₈,
halogénoalcoylthio en C₁-C₈,
3,4-difluorométhylènedioxo,
3,4-tétrafluoroéthylènedioxo,
benzyliminooxyméthyle, facultativement substitué par un groupe alcoyle en C₁-C₄, cycloalcoyle en C₃-C₆ et/ou un halogène,
cyclohexyle et cyclohexyloxy chaque fois facultativement substitués par un groupe alcoyle en C₁-C₆, alcoxy en C₁-C₆, cyclohexyle ou phényle ;
pyridyloxy facultativement substitué une ou deux fois, de manière identique ou différente, par un halogène, un groupe alcoyle en C₁-C₄ ou halogénoalcoyle en C₁-C₄ ;
phénoxy, phénylthio, benzyloxy et benzylthio, chaque fois facultativement substitués une à trois fois, de manière identique ou différente, par un groupe alcoyle en C₁-C₁₂, un halogène, un groupe halogénoalcoyle en C₁-C₄, alcoxy en C₁-C₆, halogénoalcoxy en C₁-C₆, (alcoxy en C₁-C₆)alcoyle en C₁-C₆, (alcoxy en C₁-C₆)éthylèneoxy, alcoylthio en C₁-C₆ et/ou halogénoalcoylthio en C₁-C₆ ;
D représente l'atome d'hydrogène ou un radical méthyle ;
E représente l'atome d'hydrogène ou un radical méthyle, et
G représente l'atome d'hydrogène ou un radical méthyle.

7. Oxazolines substituées de formule (Ia) suivant la revendication 6, où
A représente de préférence, un groupe alcoyle en C₁-C₆, qui est facultativement substitué une ou plusieurs fois, de manière identique ou différente, par le fluor, le chlore, le brome, un groupe cyano, alcoxy en C₁-C₂ ainsi que par phényle, phénoxy, benzyloxy, phénylthio et benzylthio, chaque fois facultativement substitués une ou deux fois, de manière identique ou différente, par le fluor, le chlore, un groupe alcoyle en C₁-C₄, halogénoalcoyle en C₁-C₂, alcoxy en C₁-C₂, halogénoalcoxy en C₁-C₂, alcoylthio en C₁-C₂ et/ou halogénoalcoylthio en C₁-C₂,
A représente également un groupe alcényle en C₂-C₆, qui est facultativement substitué une ou plusieurs fois, de manière identique ou différente, par le fluor, le chlore et/ou le brome,
A représente aussi un groupe cycloalcoyle en C₃-C₈, qui est facultativement substitué une ou plusieurs fois, de manière identique ou différente, par le fluor, le chlore, un groupe alcoyle en C₁-C₄, alcényle en C₂-C₄, ainsi que par alcoyle en C₁-C₂ ou alcényle en C₂-C₄ chaque fois substitué par le fluor et/ou le chlore ;
et A représente aussi un groupe cycloalcényle en C₅-C₇, qui est facultativement substitué une ou plusieurs fois, de manière identique ou différente, par le fluor, le chlore, un groupe alcoyle en C₁-C₄, ainsi que alcoyle en C₁-C₂ substitué par le fluor et/ou le chlore ;
B représente de préférence, un groupe phényle, benzyle, phénéth-1-yle, phénéth-2-yle, phénoxyméthyle, phénylthiométhyle, phénoxyéth-1-yle, phénoxyéth-2-yle, phénylthioéth-1-yle ou styryle, chaque fois facultativement une à quatre fois substitués, de manière identique ou différente, où l'on citera comme substituants sur les radicaux phényle :
F, Cl, Br,
alcoyle en C₁-C₁₈,
(alcoxy en C₁-C₆) alcoyle en C₁-C₈,
alcoxy en C₁-C₈ substitué une à six fois, de manière identique ou différente par F et/ou Cl,
alcoyle en C₁-C₂ substitué une à cinq fois, de manière identique ou différente par F et/ou Cl,
alcoxy en C₁-C₁₈ et -(OC₂H₄)₁₋₃-O-alcoyle en C₁C₆,
alcoylthio en C₁-C₁₂,
alcoylthio en C₁-C₈ substitué une à six fois, de manière identique ou différente par F et/ou Cl,
3,4-difluorométhylènedioxo,
3,4-tétrafluoroéthylènedioxo,
les groupements : 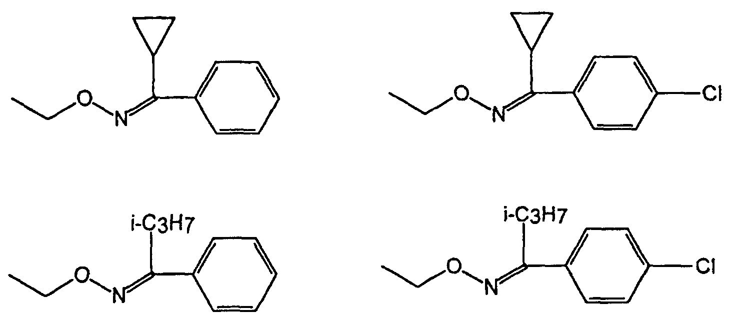
cyclohexyle et cyclohexyloxy chaque fois facultativement substitués par un groupe alcoyle en C₁C₄, alcoxy en C₁-C₄, cyclohexyle ou phényle ;
pyridyloxy facultativement substitué une ou deux fois, de manière identique ou différente, par F, Cl ou CF₃ ;
phénoxy, phénylthio, benzyloxy et benzylthio, chaque fois facultativement substitués une à trois fois, de manière identique ou différente, par alcoyle en C₁-C₁₂, F, Cl, Br, CF₃, alcoxy en C₁-C₄, alcoxy en C₁C4 substitué une à six fois, de manière identique ou différente par F et/ou Cl, (alcoxy en C₁-C₄) alcoyle en C₁-C₄, (alcoxy en C₁-C₄)éthylèneoxy, alcoylthio en C₁-C₄ et/ou alcoylthio en C₁-C₄ substitué une à six fois, de manière identique ou différente par F et/ou Cl ;
D représente l'atome d'hydrogène ou un radical méthyle ;
E représente l'atome d'hydrogène ou un radical méthyle, et
G représente l'atome d'hydrogène ou un radical méthyle.

8. Procédé de préparation d'oxazolines substituées de formule (Ia) suivant la revendication 6, **caractérisé en ce que** :
a) on fait réagir un aminoalcool de formule (II) : 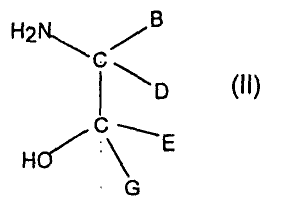 dans laquelle :
B, D, E et G ont les significations indiquées à la revendication 6,
avec un acide carboxylique de formule (III) :
A - COOH (III)
dans laquelle
A a la signification donnée à la revendication 6,
avec un agent déshydratant et le cas échéant, en présence d'un agent de dilution, ou
b) on fait réagir un amidoalcool de formule (IV) : 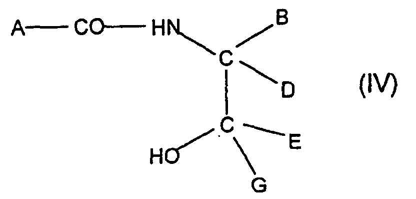 dans l'aquelle
A, B, D, E et G ont les significations indiquées ci-dessus,
avec un agent déshydratant et le cas échéant, en présence d'un agent de dilution, ou
c) on fait réagir un dérivé amide de formule (V) : 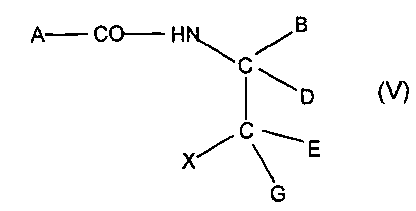 dans laquelle
A, B, D, E et G ont les significations indiquées ci-dessus, et
X représente un groupe partant,
avec une base, facultativement en présence d'un agent diluant.
